# EUROPEAN PATENT APPLICATION

(11) **EP 1 557 163 A1**
(43) Date of publication of application: **27.07.2005**
(21) Application number: 03798519.9
(22) Date of filing: 26.09.2003
(51) Int. Cl.: A61K 31/045, A61K 31/573, A61K 45/00, A61P 11/02, A61P 11/04, A61P 43/00, A61M 5/24

(54) **THERAPEUTIC CAUSING CONTRACTION OF MUCOSAL TISSUE, METHOD OF TREATING DISEASES RELATING TO MUCOSAL TISSUES, INJECTOR AND THERAPEUTIC SET**

(30) Priority: 26.09.2002 JP 2002281321
(71) Applicant: Japan Science and Technology Agency, Kawaguchi-shi, Saitama 332-0012 (JP)
(72) Inventor: WU, Men-dar, Meitou-ku, Nagoya-shi, Aichi 465-0032 (JP); INAFUKU, Shigeru, Meitou-ku, Nagoya-shi, Aichi 465-0092 (JP); KIMURA, Masaru, Tajimi-shi, Gifu 507-0064 (JP)
(74) Representative: Williams, Aylsa
(86) International application number: PCT/JP2003/012329
(87) International publication number: WO 2004/028518

(57) **Abstract**

It is intended to provide a therapeutic causing contraction of a mucosal tissue whereby various diseases relating to mucosal tissues can be easily, safely and little invasively treated, a method of treating various diseases relating to mucosal tissues with the use of the therapeutic causing contraction of a mucosal tissue, and an injector and a therapeutic set usable in the treatment method. Namely, a therapeutic causing contraction of nasal mucosal tissue containing ethanol as the active ingredient preferably together with a steroid and/or an antihistaminic agent; a method of treating diseases with mucosal inflammation using the above therapeutic causing contraction of a mucosal tissue, and an injector and a therapeutic set usable in the treatment method.

## Description

### Technical Field

The present invention relates to a therapeutic causing contraction of mucosal tissues, a method of treating various diseases relating to mucosal tissues by using the therapeutic causing contraction of mucosal tissues, an injector capable of injecting the therapeutic causing contraction of mucosal tissues, and a therapeutic set comprising the injector.

### Background Art

Conventionally, oral medicines such as an anti-allergy agent, an antihistaminic agent and a steroid preparation have been administered in one kind of treatment of nasal diseases such as hypertrophic rhinitis, vasomotor rhinitis, allergic rhinitis and nasal polyp, but a majority of these oral medicines have a transient symptomatic effect, and the symptoms occur easily repeatedly, and successive use of the oral medicine for a long time is often regarded as difficult. Further, occurrence of various side effects, for example drowsiness, dry mouth and disturbance in the liver, stomach and intestines is known. Accordingly, treatments such as nasal mucosal resection, laser ablation and cryosurgery are conducted as surgical treatment at present, and for example nasal mucosal resection is an operation where the mucosal surface in the nose is excised with an inferior turbinate scissors under local anesthesia (or general anesthesia); the laser ablation is an operation where the mucosal surface in the nose is burned with laser rays; and the cryosurgery is an operation where the mucosal surface in the nose is frozen to destroy the tissues.

However, the nasal mucosa resection, laser ablation and cryosurgery cause open and significant invasion in the mucosal surface and need a considerable time for recovery of the nasal mucosa once treated. That is, these methods are based on open ablation in the mucosal surface, and thus mucosal erosion and scab formation are caused due to the damage to the mucosal surface for a long time after the operation, so that rhinitis is adversely worsened for a long time, to give physical and mental pains and unpleasant feeling to the patient. Further, the therapeutic effect does often not reach the mucosa in a deep position where nerve fibers are actually present, and it is rare for all nasal conditions such as mucus and sneezes to be simultaneously and rapidly relieved. Re-therapy and combined use of oral medicines are often inevitable for a short time after the operation.

With respect to the effect of the surgical treatments at present (laser ablation, nasal mucosal resection, cryosurgery) on amelioration of sneezes, rhinorrhea and nasal congestion, the frequency (%) of cases where the amelioration effect occurred (excluding unchanged and deteriorated cases) is shown in Tables 1 to 3.

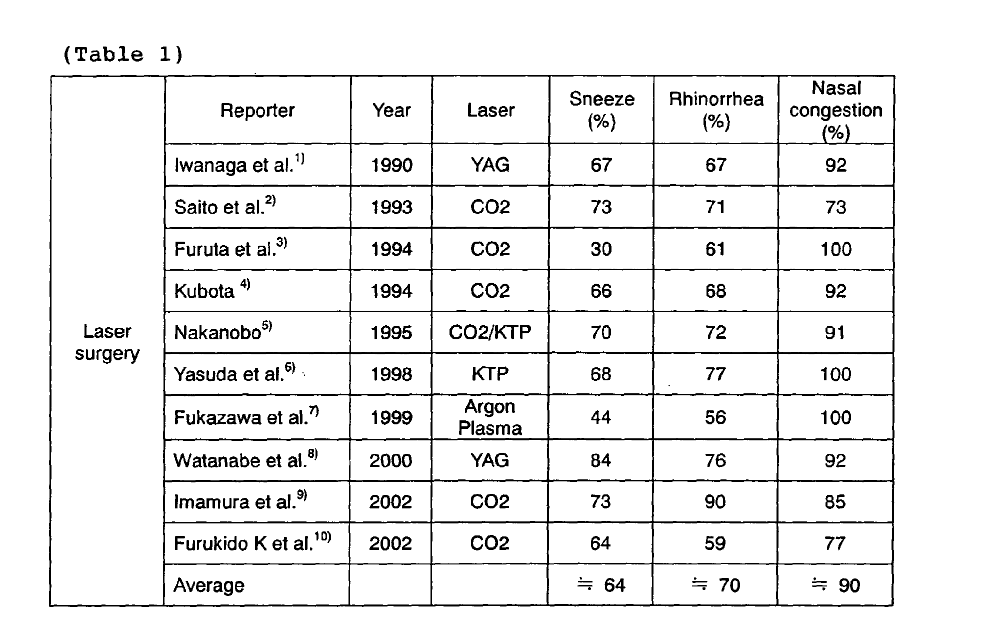

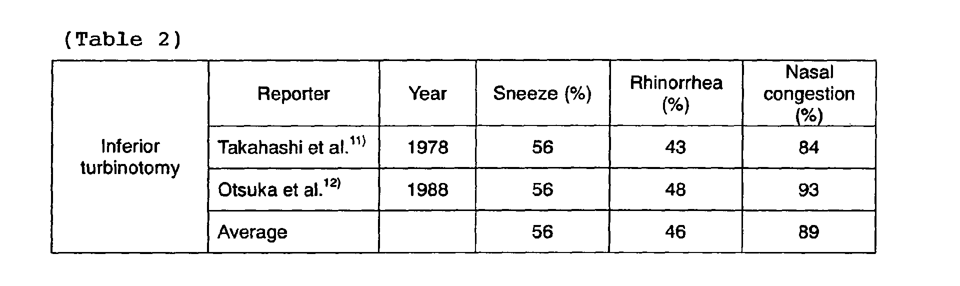

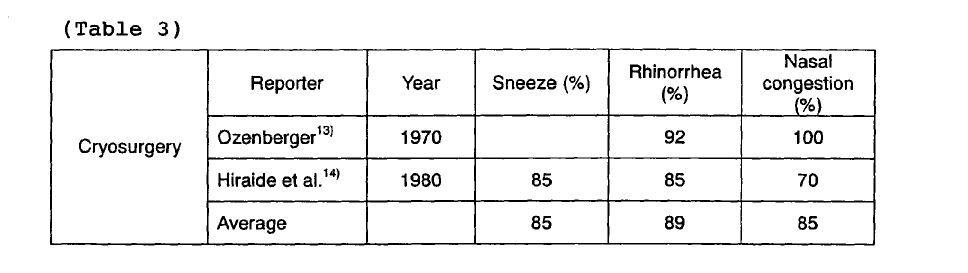

The literatures in Tables 1 to 3 are as follows:
**1)** Yasunari Iwanaga, Tadatugu Maeyama, Takemoto Shin: Surgical Treatment of Nasal Allergy with Contact Nd-YAG Laser, JIBI 36: 977-981, 1990
**2)** ShojiSaito, AtsushiArakawa, Yutaka Kitsuda, Hidenori Suzuki, Masahiro Iida: CO₂ Laser Vaporization of Nasal Mucosa in Patients with Allergic Rhinitis, JIKOTOKEI 65: 871-876, 1993
**3)** Shigeru Furuta, Koji Deguchi, Masaru Ohyama: Iatrophysics of Nasal Allergy, JOHNS 10: 389-392, 1994
**4)** Ichiyo Kubota: Laser Turbinectomy for Allergic Rhinitis, JOHNS 10: 375-381, 1993
**5)** Manabu Nakanoboh: Laser Surgery for Allergic Rhinitis, PRACT. OTOL.SUPPL. 77: 1-21, 1995
**6)** Toyotoshi Yasuda, Takashi Ishida, Ken Kitamura: Result of KTP Laser Surgery for Perennial Allergic Rhinitis, PRACT. OTOL. 91: 679-685, 1998
**7)** Keijiro Fukazawa, Hiroshi Ogasawara, Megumi Fujii, Seiichi Tomofuji, Masafumi Sakagami: Argon Plasma Surgery of the Inferior Nasal Turbinates, PRACT. OTOL. 92: 1063-1069, 1999
**8)** Akihito Watanabe, Shinichi Kawabori, Takashi Goto, Yoshiyuki Ichikawa: Contact Nd-YAG Laser Surgery for Allergic Rhinitis, PRACT. OTOL. 93: 821-826, 2000
**9)** Shunichi Imamura, Hideyuki Honda: Carbon Dioxide Laser Vaporization of Turbinate for Allergic Rhinitis, PRACT. OTOL. 95: 1037-1044, 2002
**10)** Furukido K, Takeno S, Osada R, Ishino T, Yajin K: Study of Eosinophil Activation in Nasal Musoca in Patients with Perennial Nasal Allergy: Effects of CO₂ Laser Surgery. Am J Rhinol 16: 1-6, 2002
**11)** Mitsuaki Takahashi, Minoru Okuda, Susumu Uchikoshi, Hirokuni Ohtsuka, Kosaku Sakaguchi: Global Turbinectomy of the Inferior Turbinate Mucosa for Treatment of Nasal Allergy , JIKO 50: 393-396, 1978
**12)** Hirokuni Otsuka, Yoshikiyo Sakaguchi, Takao Watase et al. : Extensive Turbinotomy for Allergic Nasal Obstruction-Long Term Efficacy-, JIKOTOKEI 60: 139-144, 1988
**13)** Ozenberger JM: Cryosurgery in chronic rhinitis. Laryngoscope 80:723-734, 1970
**14)** Fumihisa Hiraide, Masamichi Sawada, Senzo Inoue et al.: Therapeutic Experience by Cryosurgery of Allergic Rhinitis, BIFUKUBIKO 19: 158-159, 1980

The development of therapy which improves these circumstances and is easier and effective is desired, and it is expected that a high-frequency tissue reducing method such as somnoplasty and coblator surgery used actually at present as surgical treatment of obstructive sleep apnea syndrome and snoring is applied to various diseases of nasal mucosa including allergic diseases.

The high-frequency tissue reducing method is a method of coagulating and denaturing tissues by exposure to high frequency, and has been clinically used in therapy mainly for reduction of tumor tissues. It is said that the principle of the effect on reduction of tissues lies in protein coagulation by high-frequency heating. Because occurrence of protein coagulation leads inevitably to death of tissues, this method is regarded as useful in removing excessive abnormal tissues such as tumors in the living body. Focusing attention to its reducing action, Powell et al. applied its apparatus to an oral pharyngeal region for the first time in 1997 in the US. They conducted suitable exposure, to high frequency, of uvula and soft palate mucosa mainly in the cases of snore and light obstructive respiratory disturbance, to achieve an excellent ameliorating effect with respect to ablation action on mucosa and clinical symptoms (CHEST111, pp. 1348-1355, 1997; CHEST113, pp. 1163-1174, 1998).

On the basis of the report, the present inventors have also introduced a coblator system as one kind of high-frequency apparatus since spring in 2001 in Japan, to initiate clinical experiments. This therapeutic method is hardly said to be a complete method, but is an extremely useful therapeutic method depending on the case.

However, the high-frequency tissue reducing method has the problem that the cost of introducing the system and the running cost are very high. Moreover, it is actually confirmed that the surface of mucosa may be damaged for a long time after the treatment.

The present invention was made in view of such circumstances, and the object of the present invention is to provide a therapeutic causing contraction of mucosal tissues whereby various diseases relating to mucosal tissues can be easily, safely and little invasively treated, a method of treating various diseases relating to mucosal tissues with the use of the therapeutic causing contraction of mucosal tissues, and an injector and a therapeutic set usable in the treatment method.

Firstly, the present invention provides a therapeutic causing contraction of nasal mucosal tissues whereby various diseases relating to nasal mucosal tissues can be easily, safely and little invasively treated, as well as a method of treating various diseases relating to mucosal tissues with the use of the therapeutic causing contraction of nasal mucosal tissues. Secondly, the present invention provides a therapeutic causing contraction of oral pharyngeal mucosal tissues whereby various diseases relating to oral pharyngeal mucosal tissues can be easily, safely and little invasively treated, as well as a method of treating various diseases relating to mucosal tissues with the use of the therapeutic causing contraction of oral pharyngeal mucosal tissues. Thirdly, the present invention provides an injector capable of successively injecting a therapeutic easily, safely and accurately and a therapeutic set equipped with the injector.

The present inventors conceived that if the mere coagulation action on tissue protein is desired in the stage of clinical experiment of the high-frequency tissue reducing method, alcohol may be sufficient without investing very high costs for the facilities (the cost of the coblator system at that time was about ten million yen). The alcohol has a very strong coagulation action on protein, and is thus used as a fluid for fixing tissue cells in a basic field of histomorphology, and is applied clinically to a method of destructive reduction of sensory nerve nodes or tumor tissues in cancerous sharp pains or liver tumors and thyroid tumors etc. However, the idea of mucosal contraction by protein coagulation caused by injecting the alcohol directly to the submucosa has not been proposed. This is possibly due to mucosal brittleness and the damage to tissue cells caused by ethanol. That is, the killing power of ethanol which has been clinically used as a nerve blocking agent or a tumor-destroying agent, is so violent that upon direct injection of thin mucosal tissues, the problem of various complications may be caused, and thus attention has not been paid to the applicability of ethanol.

However, the present inventors believed that because the clinical effectiveness of high-frequency therapy having the same tissue-damaging mechanism as that of ethanol was confirmed, ethanol is also clinically applicable as well, and they continued the study and examined in animal experiments the effect of the tissue ablation of oral mucosa by topical injection of ethanol, to report its examination results (Journal of Otolaryngology of Japan, PROCEEDING, Vol. 105, No. 4. published on April 20, 2002). Further, the present inventors made extensive study from this report, and as a result, they found a therapeutic causing contraction of mucosal tissues whereby various diseases relating to mucosal tissues can be easily, safely and little invasively treated, a method of treating various diseases relating to mucosal tissues with the use of the therapeutic causing contraction of mucosal tissues, and an injector and a therapeutic set usable in the treatment method, and the present invention was thereby completed.

### Disclosure of the Invention

That is, the present invention relates to a therapeutic causing contraction of nasal mucosal tissues, which comprises ethanol as an active ingredient ("1"); a therapeutic causing contraction of nasal mucosal tissues, which comprises ethanol and a steroid agent and/or an antihistaminic agent as active ingredients ("2"); the therapeutic causing contraction of nasal mucosal tissues according to "1" or "2", wherein the ethanol is contained in an amount of 30 to 95% by mass ("3"); the therapeutic causing contraction of nasal mucosal tissues according to "2" or "3", wherein the steroid agent is contained in an amount of 0.01 to 1.0% by mass ("4"); the therapeutic causing contraction of nasal mucosal tissues according to any one of "2" to "4", wherein the antihistaminic agent is contained in an amount of 0.01 to 1.0% by mass ("5"); and the therapeutic causing contraction of nasal mucosal tissues according to any one of "2" to "5", which is a mixed solution of the active ingredients ("6").

Further, the present invention relates to a method of treating diseases relating to mucosal tissues, which comprises administering the therapeutic causing contraction of nasal mucosal tissues according to any one of "1" to "6" into nasal submucosa ("7"); the method of treating diseases relating to mucosal tissues according to "7", which comprises direct administration by injection into the nasal submucosa ("8"); the methodof treating diseases relating tomucosal tissues according to "8", wherein the nasal submucosa are inferior turbinate submucosa ("9"); the method of treating diseases relating to mucosal tissues according to "8", wherein the nasal submucosa is nasal polyp submucosa ("10"); the method of treating diseases relating to mucosal tissues according to any one of "8" to "10", wherein a predetermined amount of a therapeutic causing contraction of nasal mucosal tissues is injected into a plurality of sites in the nasal submucosa ("11"); the method of treating diseases relating to mucosal tissues according to "11", which comprises injection into a plurality of sites in a prickling pathway while withdrawing a needle pricked ("12"); and the method of treating diseases relating to mucosal tissues according to "11" or "12", wherein the predetermined amount is 0.05 to 5.0 mL ("13").

Further, the present invention relates to a therapeutic causing contraction of oral pharyngeal mucosal tissues, which comprises ethanol and a steroid agent and/or an antihistaminic agent as active ingredients ("14"); the therapeutic causing contraction of oral pharyngealmucosal tissues according to "14", wherein the ethanol is contained in an amount of 30 to 95% by mass ("15"); the therapeutic causing contraction of oral pharyngeal mucosal tissues according to "14" or "15", wherein the steroid agent is contained in an amount of 0.01 to 1.0% by mass ("16"); the therapeutic causing contraction of oral pharyngeal mucosal tissues according to any one of "14" to "16", wherein the antihistaminic agent is contained in an amount of 0.01 to 1.0% by mass ("17"); and the therapeutic causing contraction of oral pharyngeal mucosal tissues according to any one of "14" to "17", which is a mixed solution of the active ingredients ("18").

Further, the present invention relates to a method of treating diseases relating to mucosal tissues, which comprises administering the therapeutic causing contraction of oral pharyngeal mucosal tissues according to "14" to "18" into oral pharyngeal submucosa ("19"); the method of treating diseases relating to mucosal tissues according to "19", which comprises direct administration by injection into the oral pharyngeal submucosa ("20"); the method of treating diseases relating to mucosal tissues according to "20", wherein the oral pharyngeal submucosa is uvular submucosa, soft palate submucosa, palatopharyngeal arch submucosa, pharyngeal tonsil submucosa, palatine tonsil submucosa, lingual tonsil submucosa or lateral pharyngeal lymphatic band submucosa ("21"); the method of treating diseases relating to mucosal tissues according to "20" or "21" , wherein a predetermined amount of a therapeutic causing contraction of oral pharyngeal mucosal tissues is injected into a plurality of sites of the oral pharyngeal submucosa ("22"); the method of treating diseases relating to mucosal tissues according to "22", which comprises injection into a plurality of sites in a prickling pathway while withdrawing a needle pricked ("23"); and the method of treating diseases relating to mucosal tissues according to "22" or "23", wherein the predetermined amount is 0.05 to 5.0 mL ("24").

Further, the present invention relates to a side successive pushing injector comprising an outer cylinder, a piston capable of moving in the outer cylinder, a side projection member arranged to vigorously project from the side of the outer cylinder to the outside, a switching mechanism engaging with the piston and the side projection member, to advance the piston by pressing the side projection member toward the axis of the outer cylinder, a chemical container accommodating part arranged in the outer cylinder at the advancing side of the piston, to accommodate and retain a chemical container equipped with a bottom member capable of being pushed in a watertight state by advance of the piston, a stopper mechanism preventing return of the advanced piston, and a needle member arranged in the tip of the outer cylinder and equipped with an injection needle ("25"); the side successive pushing injector according to "25", which is used in treatment of diseases relating to mucosal tissues ("26"); the side successive pushing injector according to "25" or "26", which further comprises a temperature controlling means ("27"); the side successive pushing injector according to any one of "25" to "27", wherein a part or the whole of the injection needle is curved ("28") ; the side successive pushing injector according to "28" , wherein the direction of the tip of the injection needle, relative to the axial direction of the injection needle (the angle between the tip of the curved needle and the original position of the tip of the needle), is beyond 0° at 130° ("29"); the side successive pushing injector according to any one of "25" to "29", wherein the rear of the piston protrudes from the rear of the outer cylinder ("30"); and the side successive pushing injector according to any one of "25" to "30" , wherein the outer cylinder is provided with an opening window capable of introducing and removing the chemical container ("31").

Further, the present invention relates to a therapeutic set comprising the side successive pushing injector according to any one of "25" to "31" and a therapeutic-containing chemical container capable of being accommodated in the injector ("32"); the therapeutic set according to "32" , which is constituted such that an amount of 0.01 to 0.2 mL can be jetted out by pushing a side projection member once ("33"); the therapeutic set according to "32" or "33", wherein the chemical container has a body part, a stopper member and a bottom member, and the body part is provided with a partition wall for separating two chemicals ("34") ; the therapeutic set according to "34" , wherein a peripheral region of the partition wall is fixed to the inside of the body part, and a break induction part is formed on the surface of the partition wall ("35"); the therapeutic set according to "35", wherein the break induction part is a C-shaped linear break induction part ("36"); the therapeutic set according to any one of "34" to "36", wherein the chemical container has a pin member to break the partition wall with pushing ("37"); the therapeutic set according to any one of "34" to "37" , wherein the partition wall is arranged in the vicinity of the stopper member ("38"); the therapeutic set according to "32" or "33", wherein the chemical container has a body part, a stopper member and a bottom member, and a chemical accommodating chamber is arranged in the stopper member ("39"); the therapeutic set according to "39", wherein a supporting member is arranged in the bottom of the chemical accommodating chamber, and a break induction part surrounding the supporting member is formed in the bottom of the chemical accommodating chamber ("40"); the therapeutic set according to "40", wherein the break induction part is a C-shaped linear break induction part ("41"); the therapeutic set according to any one of "39" to "41", wherein an injection needle in an injection needle member is arranged with eccentricity, and upon fitting of the needle member to an outer cylinder, the bottom of the chemical accommodating chamber is broken by pressing with the rear end of the injection needle against it ("42") ; the therapeutic set according to "32" or "33" , wherein the therapeutic in the chemical container is the therapeutic causing contraction of nasal mucosal tissues according to any one of "1" to "6" ("43"); the therapeutic set according to "32" or "33", wherein the therapeutic in the chemical container is the therapeutic causing contraction of oral pharyngeal tissues according to any one of "13" to "17" ("44"); the therapeutic set according to "34" to "42", wherein the active ingredients in the therapeutic causing contraction of nasal mucosal tissues according to any one of "2" to "5" are accommodated separately ("45"); and the therapeutic set according to "34" to "42", wherein the active ingredients in the therapeutic causing contraction of oral pharyngeal mucosal tissues according to any one of "13" to "16" are accommodated separately ("46").

### Brief Description of Drawings

Fig. 1 is a schematic illustration showing the working mechanism, on mucosal tissues, of the method of treating diseases relating to mucosal tissues by using the therapeutic causing contraction of nasal mucosal tissues according to the present invention.
Fig. 2(A) is a schematic illustration of an injector according to the first embodiment of the present invention; Fig. 2(B) is a sectional view along 1B-1B of the injector shown in Fig. 2(A).
Fig. 3 is a set of illustrations showing the movement of a piston in the injector shown in Fig. 2.
Fig. 4 is an illustration of the mechanism of injection of a therapeutic by push of the piston in the injector shown in Fig. 2.
Fig. 5(A) is a schematic illustration of the injector according to the second embodiment of the present invention, and Fig. 5(B) is a sectional view along 4B-4B of the injector shown in Fig. 5(A).
Fig. 6 is a set of illustrations showing the movement of a piston in the injector shown in Fig. 5.
Fig. 7 is a schematic illustration of the injector according to the third embodiment of the present invention.
Fig. 8 is a perspective view of a chemical container accommodated in the injector shown in Fig. 7.
Fig. 9 is a set of illustrations showing use of the injector shown in Fig. 7.
Fig. 10 is a schematic illustration of the injector according to the fourth embodiment of the present invention.
Fig. 11 is a perspective view of a chemical container accommodated in the injector shown in Fig. 10.
Fig. 12 is a set of illustrations showing use of the injector shown in Fig. 10.
Fig. 13 is a partially enlarged view of the injector shown in Fig. 12.
Fig. 14 is a schematic illustration of the injector according to the fifth embodiment of the present invention.
Fig. 15 is a set of illustrations showing use of the injector shown in Fig. 14.
Fig. 16 is a set of illustrations showing use of the injector shown in Fig. 14.
Fig. 17 is a set of photographs showing the observation result of the right inferior turbinate mucosal tissues by a fiber scope before and after the ethanol treatment in Example 1-1 in the present invention.
Fig. 18 is a set of photographs showing the observation result of the left inferior turbinate mucosal tissues before and after the control treatment in Comparative Example 1-1.
Fig. 19 is a graph showing the evaluation result based on the evaluation criteria in Table 4 with time after ethanol treatment and control treatment of the patient.
Fig. 20 is a set of photographs showing the observation result of the left inferior turbinate mucosal tissues by a fiber scope before and after the treatment in Example 1-2 in the present invention.
Fig. 21 is a set of photographs showing the observation result of the right inferior turbinate mucosal tissues by a fiber scope before and after the treatment in Example 1-2 in the present invention.
Fig. 22 is a graph showing the evaluation result based on the evaluation criteria in Table 4 with time after the treatment of the patient in Example 1-2.
Fig. 23 is a set of photographs showing the observation result of the left inferior turbinate mucosal tissues by a fiber scope before and after the ethanol treatment in Example 1-4 in the present invention.
Fig. 24 is a set of photographs showing the observation result of the left inferior turbinate mucosal tissues by a fiber scope before and after the steroid-containing treatment in Example 1-4 in the present invention.
Fig. 25 is a graph showing the degree of invasion (mean) into the inferior turbinate mucosa with time in 3 patients subjected to the ethanol treatment and steroid-containing treatment in Example 1-5 in the present invention.
Fig. 26 is a set of photograph showing the observation result of the inferior turbinate mucosal tissues by a fiber scope before and after the treatment in Example 1-6 in the present invention.
Fig. 27 is a photograph showing the result of histopathological examination of the inferior turbinate mucosal tissues before the treatment in Example 1-6 in the present invention.
Fig. 28 is a set of photographs showing the observation result of the inferior turbinate mucosal tissues by a fiber scope before and after the treatment in Example 1-7 in the present invention.
Fig. 29 is a set of photographs showing the result of histopathological examination of the inferior turbinate mucosal tissues before and after the treatment in Example 1-7 in the present invention.
Fig. 30 is a set of photographs showing the observation result of uvular mucosal tissues with a fundus camera upon an ocular inspection of the oral cavity before and after the treatment in Example 2-1 in the present invention.
Fig. 31 is set of photographs showing the observation result of uvular mucosal tissues with a fundus camera upon an ocular inspection of the oral cavity before and after the treatment in Example 2-1 in the present invention.
Fig. 32 is a set of photographs showing the observation result of uvular mucosal tissues with a fundus camera upon an ocular inspection of the oral cavity before and after the treatment in Example 2-1 in the present invention.
Fig. 33 is a set of photographs showing the observation result with a fundus camera or fiber scope upon an ocular inspection of the oral cavity before and after the treatment (6 months after the first treatment), the result of cephalometric radiogram (cephalometry), and the results of AHI and VSA in Example 2-1 in the present invention.
Fig. 34 is a set of photographs showing the observation result of uvular mucosal tissues with a fundus camera upon an ocular inspection of the oral cavity before and after the treatment in Example 2-2 in the present invention.
Fig. 35 is a graph showing a change in VAS before and after the treatment with the therapeutic in Example 2-3 in the present invention.
Fig. 36 is a graph showing a change in the length of the uvula before and after the treatment with the therapeutic in Example 2-3 in the present invention.
Fig. 37 is a set of photographs showing the observation result of palatine tonsil mucosal tissues with a fundus camera or fiber scope upon an ocular inspection of the oral cavity before and after the treatment in Example 2-4 in the present invention.
Fig. 38 is a set of photographs showing the observation result of uvular mucosal tissues with a fundus camera upon an ocular inspection of the oral cavity before and after the treatment in Comparative Example 2-1 in the present invention.

### Best Mode of Carrying Out the Invention

The therapeutic causing contraction of nasal mucosal tissues according to the present invention comprises ethanol as the active ingredient. The nasal submucosa to which the therapeutic causing contraction of nasal mucosal tissues according to the present invention is applied includes, for example, inferior turbinate submucosa, middle turbinate submucosa, and nasal polyp submucosa, particularly preferably inferior turbinate submucosa. Particularly, the turbinate submucosa is rich in nasal glands, a vascular network showing a sponge-like structure, and sensory and autonomous nerve fibers , and is generally useful for air-conditioning action in the nasal cavity and for a change in physiological dilation, and under morbid states such as allergic rhinitis, hypertension of parasympathetic nervous system under the mucosa leads easily to expansion of the vascular network and promotion of the nasal gland secreting function, thus frequently causing dilation of nasal mucosa and excessive secretion of mucus, and hypersensitivity of trigeminal nerve as sensory nerve further excites a sneeze nerve reflex arch formed via brain stem reticular formation between trigeminal nerve and motor nerve system such as glossopharyngeal nerve, vagus nerve and phrenic nerve, to cause frequent sneezes, but when the therapeutic causing contraction of nasal mucosal tissues according to the present invention is applied, it becomes possible to reduce nasal congestion by contraction and reduction of mucosa, to reduce secretion of mucus, to reduce mucosal congestive dilation and to suppress a spasm of sneezing by damaging or partially destroying nasal glands, vascular network and nerve fibers such as parasympathetic nerves and trigeminal nerves simultaneously by the coagulation action of ethanol.

The content of ethanol in the therapeutic causing contraction of nasal mucosal tissues according to the present invention is preferably 30 to 95% by mass, more preferably 50 to 80% by mass, still more preferably 60 to 75% by mass, further more preferably 65 to 70% by mass. When the ethanol content is in the above range, suitable contraction of mucosal tissues is made feasible by administering a suitable amount of the therapeutic, to contract the mucosal tissues suitably without giving excessive burden on the tissues.

In the therapeutic causing contraction of nasal mucosal tissues according to the present invention, a steroid agent is preferably contained as the active ingredient, and the content of the steroid agent in the therapeutic is preferably 0.01 to 1.0% by mass, more preferably 0.05 to 0.5% by mass, still more preferably 0.08 to 0.4% by mass, further more preferably 0.1 to 0.3% by mass. The unspecific inflammatory infiltration and expansion of topical mucosa accompanying the denaturation of mucosa with ethanol can be minimized by the anti-inflammatory action and anti-edema action of the steroid agent, and this effect is extremely significant as illustrated in the Examples described later. Accordingly, topical sharp pain and unpleasant feeling caused by inflammatory reaction of mucosa after administration of the therapeutic would be reduced. This steroid agent is easily dissolved in ethanol, and from related data on extraction from tissues, it is considered that the mutual pharmacological action of the mixed alcohol and steroid does not cause not only changes in outward appearance but also biochemical changes (the Pharmaceutical Affairs Bureau of the Ministry of Health and Welfare, Second Evaluation Division: The Japanese Pharmacopoeia, Non-Pharmaceutical Preparation, the Ministry of Health and Welfare P-P: 1282-1284, 1989, "Steroid Hormone, Hormone II Non-Peptide Hormone, New Lecture Experimental Biochemistry 9 (in Japanese)" edited by the Japanese Biochemical Society, Tokyo Kagaku Dojin P-P:81-109, 1992), and even if the steroid agent is contained in the therapeutic comprising ethanol as the active ingredient according to the present invention, its effect can be sufficiently demonstrated.

Specifically, the steroid agent is preferably the one having a high anti-inflammatory titer, and examples include sodium dexamethasone phosphate, sodium betamethasone phosphate, sodium prednisolone phosphate, methyl prednisolone acetate, sodium prednisolone succinate, dexamethasone acetate, betamethasone acetate/sodium betamethasone phosphate, triamcinolone acetonide etc., among which sodium dexamethasone phosphate and sodium betamethasone phosphate are preferable in respect of high titer of anti-inflammatory action and lower reaction to foreign matter. Specific examples include Decadron^{R}, Orgadron^{R}, Rinderon^{R}, Corson^{R} etc. among which Orgadron^{R} and Rinderon^{R} are preferable in respect of solubility.

The therapeutic causing contraction of nasal mucosal tissues according to the present invention preferably contains an antihistaminic agent as the active ingredient, and the content of the anti-histaminic agent in the therapeutic is preferably 0.01 to 1.0% by mass, more preferably 0.05 to 0.5% by mass, still more preferably 0.1 to 0.3% by mass. By incorporation of the antihistaminic agent, functions of histamine, that is, actions such as expansion of peripheral blood vessels, promotion of permeation of blood vessel walls and promotion of secretion in gland tissues are strongly inhibited, and thus the antihistaminic agent is effective for inflammatory reaction in topical mucosa by stimulation with ethanol injected, especially transient excessive secretion of a tissue exudate and reduction in edema. Specifically, the antihistaminic injection includes diphenhydramine hydrochloride, chlorpheniramine maleate (dl-configuration, d-configuration), diphenylpyraline teoclate, diphenylpyraline hydrochloride, promethazine hydrochloride etc., and examples include Resmin^{R}, Chlorotrimeton^{R}, Polaramine^{R}, Procon^{R}, Hy-stamine^{R} etc. Resmin^{R} and Polaramine^{R} are preferable because they are easily dissolved in ethanol and have high titer of antihistamine and low anti-choline action and low inhibitory action on the central nervous system.

In the therapeutic causing contraction of nasal mucosal tissues according to the present invention, the active ingredients (ethanol, steroid agent, antihistaminic agent) may be accommodated respectively in containers or the like or may be accommodated as a mixed solution in a container or the like.

From the difference in reactivity of ethanol to nasal mucosa and oral pharyngeal mucosa, the steroid agent and/or antihistaminic agent is not necessarily be contained as the active ingredient in the therapeutic causing contraction of nasal mucosal tissues according to the present invention. That is, swelling of the oral pharyngeal mucosa, even in a moderate or less degree, can cause blockage of the upper respiratory tract in the pharyngeal cavity and leads directly to unexpected accidents such as suffocation, and thus simultaneous use of the steroid agent and the antihistaminic agent is necessary, while in the case of the nasal mucosa, it was revealed that the degree of dilation of topical mucosa caused by injection of the same volume of ethanol can be suppressed relatively lightly, and it is hardly estimated that under the condition where both nasal cavities are almost closed and do not function as a respiratory pathway due to the original morbid swelling of nasal mucosa, further swelling of mucosa after the injection causes a pain in breathing.

If necessary, the therapeutic causing contraction of nasal mucosal tissues according to the present invention may contain a suitable amount of an anesthetic and a vascular shrinking agent preventing diffusion of ethanol. Specific examples of the anesthetic include lidocaine, lidocaine hydrochloride, mepivacaine hydrochloride, propitocaine hydrochloride, procaine hydrochloride, tetocaine hydrochloride etc. The vascular shrinking agent includes epinephrine etc. Particularly, a combination of an anesthetic and a vascular shrinking agent, for example Xylocaine^{R} (E injection) and Citanest^{R} (E injection), is used more preferably.

The therapeutic causing contraction of nasal mucosal tissues according to the present invention can contract nasal mucosal tissues, broaden the nasal cavity to improve nasal breathing, and can, easily and safely with minimum invasion, ameliorate symptoms of nasal congestion caused by chronic hypertrophic rhinitis, vasomotor rhinitis, perennial or seasonal allergic rhinitis, chronic sinusitis, and nasal polyp, and can suppress a reflex arch of submucosal nerve fibers, and thus a spasm of sneezing and mucus secretion caused by stimulative reflex of nerves can be ameliorated. Further, it comprises ethanol as the active ingredient, and is thus extremely inexpensive, highly safe and scarcely causes side effects and aftereffects on humans. In addition, when the therapeutic causing contraction of nasal mucosal tissues according to the present invention, comprising additives such as the steroid agent, antihistaminic agent, anesthetic and vascular shrinking agent, is administered by injection, an open wound is not formed after injection, and the leakage of the injected therapeutic hardly occurs, and thus the additives such as steroid agent etc. are retained very efficiently in the tissues, and their action can be exhibited to the maximum degree, and the content of the additives can be further reduced.

Then, the method of treating diseases relating to mucosal tissues in the nasal cavity according to the present invention is described.

The method of treating diseases relating to mucosal tissues in the nasal cavity according to the present invention comprises administering the therapeutic causing contraction of nasal mucosal tissues into the nasal submucosa. In the method of administering the therapeutic for mucosal tissues, the therapeutic causing contraction of mucosal tissues can be injected directly into the nasal submucosa, and as an injector, a general injector can be used, and the side successive pushing injector of the present invention described later is preferably used because the therapeutic can be injected easily, safely and accurately. The nasal submucosa into which the therapeutic causing contraction of nasal mucosal tissues according to the present invention is administered includes, for example, inferior turbinate submucosa, middle turbinate submucosa, and nasal polyp submucosa, particularly preferably inferior turbinate submucosa. Particularly, the turbinate submucosa is rich in nasal glands, a vascular network showing a sponge-like structure, and sensory and autonomous nerve fibers, and is generally useful for air-conditioning action in the nasal cavity and for a change in physiological swelling, and under morbid states such as allergic rhinitis, hypertension of parasympathetic nervous system in the mucosa leads easily to dilatation of the vascular network and an acceleration of the nasal gland secreting function, thus frequently causing swelling of nasal mucosa and excessive secretion of mucus, and hypersensitivity of trigeminal nerve as sensory nerve further excites a sneeze nerve reflex arch formed via brain stem reticular formation between trigeminal nerve and motor nerve system such as glossopharyngeal nerve, vagus nerve and phrenic nerve, to cause frequent sneezes, but when the method of treating diseases relating to mucosal tissues according to the present invention is applied, it becomes possible to reduce nasal congestion by contraction and reduction of mucosa, to reduce secretion of mucus, to reduce mucosal congestive swelling and to suppress a spasm of sneezing by damaging or partially destroying nasal glands, vascular network and nerve fibers such as parasympathetic nerves and trigeminal nerves simultaneously by the coagulation action of ethanol.

The dose of the therapeutic for mucosal tissues can be suitably determined depending on the state of lesions and the severity of conditions, and in consideration of the expansion of submucosa, the amount of the therapeutic administered is generally preferably 0.05 to 2.0 mL, more preferably 0.1 to 1.0 mL. The therapeutic for mucosal tissues may be administered all at once, but is preferably injected into a plurality of sites to prevent a rapid change in tissues and treat the tissues more safely, and it is more preferable that the tip of a needle is inserted into submucosa, and while withdrawing the needle, the position of the tip of the needle is changed in the submucosa to inject the therapeutic into a plurality of sites in the prickling pathway. When the therapeutic is administered in divided portions, each portion is preferably 0.01 to 0.5 ml, more preferably 0.05 to 0.3 ml, still more preferably 0.08 to 0.15 ml. Particularly when the ethanol concentration is high, topical denaturation and swelling occur more rapidly, and thus the therapeutic is administered preferably into a plurality of site while the injection volume per site is decreased.

When the therapeutic to be administered in the method of treating diseases relating to mucosal tissues in the nasal cavity according to the present invention does not contain a steroid agent and/or an antihistaminic agent, a steroid agent and/or an antihistaminic agent may be administered before and/or after the administration of the therapeutic. Before injection, mucosal tissues to be treated are preferably subjected to surface anesthesia or infiltration anesthesia with an anesthetic. Basically, anesthesia may be local anesthesia, and general anesthesia is not necessary, thus facilitating treatment. After injection, hospitalization or medical treatment is not necessary, and thus the surgery can be sufficiently finished in one day without hospitalization.

Themethodof treating various diseases relating tomucosal tissues in the nasal cavity according to the present invention can contract nasal mucosal tissues, broaden the nasal cavity to improve nasal breathing, and can, easily and safely with minimum invasion, ameliorate symptoms of nasal congestion caused by chronic hypertrophic rhinitis, vasomotor rhinitis, perennial or seasonal allergic rhinitis, chronic sinusitis, and nasal polyp, and can suppress a reflex arch of submucosal nerve fibers, and thus a spasm of sneezing and mucus secretion caused by stimulative reflex of nerves can be ameliorated. Further, the method of treating various diseases relating to mucosal tissues in the nasal cavity according to the present invention does not involve procedures such as cutting, excision or ablation directly on the surface of the nasal mucosa so that after the treatment, formation of ulcer and scab on the nasal mucosa, nosebleed, increase of contaminated rhinorrhea and complications of infections in the nasal cavity can be minimized, and physical burden on the patient can be reduced. In the method of treating various diseases relating to mucosal tissues in the nasal cavity according to the present invention, the therapeutic comprising ethanol as the active ingredient for construction of mucosa is used, and is thus extremely inexpensive, highly safe and scarcely causes side effects and aftereffects on humans. In the method of treating various diseases relating to mucosal tissues in the nasal cavity according to the present invention, an open wound is not formedafter administration (injection) of the therapeutic, and the leakage of the injected therapeutic hardly occurs so that when the therapeutic contains additives such as the steroid agent, antihistaminic agent, anesthetic and vascular shrinking agent, the additives such as steroid agent etc. are retained very efficiently in the tissues, and their action can be exhibited to the maximum degree, and the content of the additives can be further reduced.

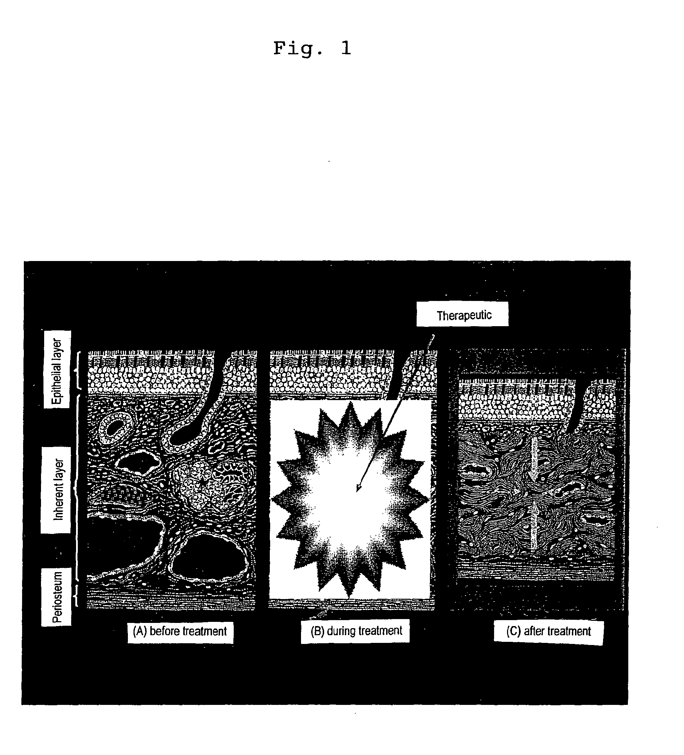

Hereinafter, the action, on mucosal tissues, of the method of treating diseases relating to mucosal tissues by using the therapeutic causing contraction of nasal mucosal tissues according to the present invention is described by reference to the Drawings. Fig. 1 is a schematic illustration showing the working mechanism, on mucosal tissues, of the method of treating diseases relating to mucosal tissues by using the therapeutic causing contraction of nasal mucosal tissues according to the present invention, wherein (A) is before treatment, (B) is during treatment, and (C) is after treatment.

As shown in Fig. 1 (A) to (C), the nasal mucosa, particularly inferior turbinate mucosa has the lamina propria (submucosa) on the periosteum, and the lamina propria is covered with a mucosal epithelial layer.

Nasal glands and autonomic nerve fibers relating to secretion of nasal mucus, sensory nerve fibers relating to reflex of sneezes, and a sponge-like vascular plexus relating significantly to reactive swelling of mucosa (nasal congestion) are distributed mainly in the lamina propria, and accordingly the occurrence of various conditions (rhinorrhea, sneezes, nasal congestion etc.) of rhinitis, particularly allergic rhinitis, is significantly related to the morbid state of the submucosa. That is, it can be said that the regulation of change in the morbid state of the lamina propria is directly connected with improvement of various conditions of rhinitis. The mucosal epithelial layer plays an important role in exhibiting physiological functions in the nasal cavity, such as elimination of microorganisms and dust by filtration and protection against local infections. Accordingly, there is need for a therapeutic method by which only the submucosal tissues as the central lesion of rhinitis can be treated while the mucosal epithelial is maintained intact, but the conventional laser ablation surgery is a therapeutic method that completely removes both the lamina propria and epithelial layer.

In the method of treating diseases relating to mucosal tissues by using the therapeutic causing contraction of nasal mucosal tissues according to the present invention, as shown in Fig. 1(B), the therapeutic can be administered directly into the lamina propria, and thus only the lamina propria where nasal glands, nerve fibers and vascular plexus are present in a large amount can be easily and very efficiently damaged or partially destroyed while damage to the mucosal epithelial layer is minimized, resulting in reduction in excess rhinorrhea, reduction in induction of sneezes and reduction in vascular plexus, and the damaged or partially destroyed submucosa become fibrous, and as shown in Fig. 1(C), the volume of the mucosa is reduced due to the cicatrix shrinkage, and ameliorating nasal congestion by calming the mucosal swelling.

As described above, the therapeutic method of treating diseases relating to mucosal tissues by using the therapeutic causing contraction of nasal mucosal tissues according to the present invention exhibits a significantly outstanding effect by administering the therapeutic into the submucosa whereby the submucosa is contracted without damaging the mucosal epithelial layer, with the cicatrix by formation of fibers, and recover the normal state from the swollen and enlarged mucosal tissues.

The therapeutic causing contraction of oral pharyngeal mucosal tissues as described later has the same mechanism as in the nasal mucosa in respect of attaining an effect of reduction by cicatrix shrinkage of the lamina propria of the oral mucosa, but nerve fibers, secretion glands and vascular plexus occur in a higher amount in the nasal mucosa than in the oral mucosa, so that very significant effects not previously anticipated, such as reduction in denaturation of nerve fibers and damage and destruction of secretory glands and vascular plexus, having a very important meaning similar to a reduction in tissues, can be achieved.

Now, the therapeutic causing contraction of oral pharyngeal mucosal tissues according to the present invention is described.

The therapeutic causing contraction of oral pharyngeal mucosal tissues according to the present invention comprises ethanol and a steroid agent and/or an antihistaminic agent as active ingredients. The oral or pharyngeal submucosa to which the therapeutic causing contraction of oral pharyngeal mucosal tissues according to the present invention is applied includes, for example, submucosa such as uvular submucosa, soft palate submucosa, platoglossal arch (anterior palatine arch) submucosa, platopharyngeal arch (posterior palatine arch) submucosa, radix linguae submucosa, tissues under Waldeyer pharyngeal ring submucosal lymph tissues (pharyngeal tonsil tissues, palatine tonsil tissues, lingual tonsil tissues, pharyngeal lateral lymph follicles), particularly preferably uvular submucosa, soft palate submucosa, platopharyngeal arch submucosa, pharyngeal tonsil submucosa, palatine tonsil submucosa, lingual tonsil submucosa or pharyngeal lateral submucosa. The submucosa includes not only the lamina propria of mucosal tissues but also its lower layer such as muscular layer.

Snoring and obstructive sleep apnea syndrome are due to soft palate vibrational snoring or soft palate stenotic respiratory disturbance, and the oral pharyngeal finding is characterized mainly by excessive growth of the uvula, thickening and lower position of the soft palate, growth of the width of the palatopharyngeal arch and enlargement of tonsils, and thus the injection of the therapeutic into these sites is extremely effective in reduction in vibrating sound by formation of mucosal cicatrix shrinkage at that site, expansion of air way in the oral narrow region, and prevention of sinking of uvula/soft palate caused by tissue hardening, resulting in significant amelioration of snoring and obstructive sleep apnea syndrome. For Waldeyer's tonsillar ring, particularly the tissue of palatine tonsil and lymph follicles in the lateral pharyngeal lymphatic band, which have main inflammatory tissue lesion, the contractive hardening effects due to coagulation of tonsillar tissues by injection of the therapeutic leads to reduction and stabilization of inflamed tonsillar tissues, and seems to effectively contribute to the prevention of enlargement and calming of inflammatory reaction in these tissues.

In the therapeutic causing contraction of oral pharyngeal mucosal tissues according to the present invention, the content of ethanol in the therapeutic is preferably 30 to 95% by mass, more preferably 50 to 80% by mass, still more preferably 60 to 75% by mass, further more preferably 65 to 70% by mass. When the ethanol content is in the above range, suitable contraction of mucosal tissues is made feasible by administering a suitable amount of the therapeutic, to contract the mucosal tissues suitably without giving excessive burden on the tissues.

The content of the steroid agent in the therapeutic is preferably 0.01 to 1% by mass, more preferably 0.05 to 0.5% by mass, still more preferably 0.08 to 0.4% by mass, further more preferably 0.1 to 0.3% by mass. The unspecific inflammatory infiltration and swelling of topical mucosa accompanying the denaturation of mucosa with ethanol can be minimized by the anti-inflammatory action and anti-edema action of the steroid agent, and this effect is extremely significant as illustrated in the Examples later. Accordingly, topical sharp pain and unpleasant feeling caused by inflammatory reaction of mucosa after administration of the therapeutic would be reduced. This steroid agent is easily dissolved in ethanol, and from related data on extraction from tissues, it is considered that the mutual pharmacological action of the mixed alcohol and steroid does not cause not only changes in outward appearance but also biochemical changes (the Pharmaceutical Affairs Bureau of the Ministry of Health and Welfare, Second Evaluation Division: The Japanese Pharmacopoeia, Non-Pharmaceutical Preparation, the Ministry of Health and Welfare P-P:1282-1284, 1989, "Steroid Hormone, Hormone II Non-Peptide Hormone, New Lecture Experimental Biochemistry 9 (in Japanese) " edited by the Japanese Biochemical Society, Tokyo Kagaku Dojin P-P:81-109, 1992), and even if the steroid agent is contained in the therapeutic comprising ethanol according to the present invention, its effect can be sufficiently demonstrated.

Specifically, a topical injection of the steroid agent is preferably the one having a high anti-inflammatory titer, and examples include sodium dexamethasone phosphate, sodium betamethasone phosphate, sodium prednisolone phosphate, methyl prednisolone acetate, sodium prednisolone succinate, dexamethasone acetate, betamethasone acetate/sodium betamethasone phosphate, triamcinolone acetonide etc., among which sodium dexamethasone phosphate and sodium betamethasone phosphate are preferable in respect of high titer of anti-inflammatory action and lower reactivity as a foreignmatter. Specific examples include Decadron^{R}, Orgadron^{R}, Rinderon^{R}, Corson^{R} etc. among which Orgadron^{R} and Rinderon^{R} are preferable in respect of solubility.

The content of the anti-histaminic agent in the therapeutic is preferably 0.01 to 1% by mass, more preferably 0.05 to 0.5% by mass, still more preferably 0.1 to 0.3% by mass. By incorporation of the antihistaminic agent, functions of histamine, that is, actions such as dilatation of peripheral blood vessels, promotion of permeation of blood vessel walls and promotion of secretion in glandular tissues are strongly inhibited, and thus the antihistaminic agent is effective for inflammatory reaction in topical mucosa by stimulation with ethanol injected, especially reduction in edema. Specifically, the antihistaminic agent includes diphenhydramine hydrochloride, chlorpheniramine maleate (dl-configuration, d-configuration), diphenylpyraline teoclate, diphenylpyraline hydrochloride, promethazine hydrochloride etc., and examples include Resmin^{R}, Chlorotrimeton^{R}, Polaramine^{R}, Procon^{R}, Hy-stamine^{R} etc. Resmin^{R} and Polaramine^{R} are preferable because they are easily dissolved in ethanol and have high titer of antihistamine and low anti-choline action and low inhibitory action on the central nervous system.

In the therapeutic causing contraction of oral pharyngeal mucosal tissues according to the present invention, the active ingredients (ethanol, steroid agent, antihistaminic agent) may be accommodated respectively in containers or the like or may be accommodated as a mixed solution in a container or the like.

In the case of oral pharyngeal mucosa, a difficulty in eating due to a sharp pain after injection or the stenosis of the upper respiratory tract due to swelling of mucosal tissues occurs, and a difficulty in respiration may be caused, and swelling of the mucosa even in a moderate or less degree can cause blockage of the upper respiratory tract in the pharyngeal cavity and leads directly to unexpected accidents such as suffocation, and thus the steroid agent and/or antihistaminic agent should be contained therein.

If necessary, the therapeutic causing contraction of oral pharyngeal mucosal tissues according to the present invention may contain a suitable amount of an anesthetic and a vasoconstrictor preventing diffusion of ethanol. Specific examples of the anesthetic include lidocaine, lidocaine hydrochloride, mepivacaine hydrochloride, propitocaine hydrochloride, procaine hydrochloride, tetocaine hydrochloride etc. The vasoconstrictor includes epinephrine etc. Particularly, a combination of an anesthetic and a vasoconstrictor, for example Xylocaine^{R} (E injection) and Citanest^{R} (E injection), is used more preferably.

The therapeutic causing contraction of oral pharyngeal mucosal tissues according to the present invention can contract mucosal tissues while minimizing unspecific inflammatory infiltration and swelling of the mucosal tissues, to ameliorate snoring and obstructive sleep apnea syndrome easily and safely with minimum invasion. Further, ethanol contained in the therapeutic causing contraction of oral pharyngeal mucosal tissues according to the present invention is extremely inexpensive, highly safe and scarcely causes side effects and aftereffects on humans. In addition, when the therapeutic causing contraction of oral pharyngeal mucosal tissues according to the present invention is administered by injection, an open wound is not formed after injection, and the leakage of the injected therapeutic hardly occurs, and thus the steroid agent is retained very efficiently in the tissues, and its action can be exhibited to the maximum degree, and the content of the steroid agent can be further reduced. When additives such as the above-mentioned antihistaminic agent, anesthetic and vasoconstrictor are contained, their action can be exhibited to the maximum degree, and the content of the additives can be further reduced.

Then, the method of treating diseases relating to oral or pharyngeal mucosal tissues according to the present invention is described.

The method of treating diseases relating to oral or pharyngeal mucosal tissues according to the present invention comprises administering the therapeutic causing contraction of oral or pharyngeal mucosal tissues into the oral or pharyngeal submucosa. In the method of administering the therapeutic causing contraction of oral or pharyngeal mucosal tissues, the therapeutic causing contraction of oral or pharyngeal mucosal tissues can be administered directly by injection into the oral or pharyngeal submucosa, and as an injector, a general injector can be used, and the side successive pushing injector of the present invention described later is preferably used because the therapeutic can be injected easily, safely and accurately. The oral or pharyngeal submucosa into which the therapeutic causing contraction of oral or pharyngeal mucosal tissues according to the present invention is administered includes, for example, submucosa such as uvular submucosa, soft palate submucosa, palatoglossal arch (anterior palatine arch) submucosa, platopharyngeal arch (posterior palatine arch) submucosa, radix linguae submucosa, submucosal lymphatic tissues of Waldeyer' s tonsillar ring (pharyngeal tonsil tissues , palatine tonsil tissues, lingual tonsil tissues, lateral pharyngeal lymphatic band), particularly preferably uvular submucosa, soft palate submucosa, platopharyngeal arch submucosa, pharyngeal tonsil submucosa, palatine tonsil submucosa, lingual tonsil submucosa or lateral pharyngeal lymphatic band tissues. The submucosa includes not only the lamina propria but also its lower tissues such as muscular layer.

Snoring and obstructive sleep apnea syndrome are due to soft palate vibrational snoring or soft palate constrictive respiratory disturbance, and the oral pharyngeal finding is characterizedmainlyby excessive growth of the uvula, thickening and lower position of the soft palate, growth or elongation of the width of the palatine pharyngeal arch and enlargement of tonsils, and thus the injection of the therapeutic into these sites is extremely effective in reduction in vibrating sound by formation of mucosal cicatrix shrinkage at that site, expansion of air way in the oral narrow region, and prevention of sinking of uvula/soft palate caused by tissue hardening, resulting in significant amelioration of snoring and obstructive sleep apnea syndrome. For Waldeyer's lymphatic ring having a main lesion, particularly palatine tonsil tissues and lateral pharyngeal lymph follicles particularly in chronic tonsillitis and focal infection among inflammatory diseases in the oral pharyngeal mucosa, the contractive hardening effects due to coagulation of tonsillar tissues by injection of the therapeutic leads to reduction and stabilization of inflamed tonsillar tissues, and seems to effectively contribute to the prevention of enlargement and calming of inflammatory reaction in these tissues.

The dose of the therapeutic causing contraction of oral pharyngeal mucosal tissues according to the present invention can be suitably determined depending on the state of lesions and the severity of conditions, and in consideration of the swelling of submucosa, the dose is generally preferably 0.05 to 2 mL, more preferably 0.1 to 1.0 mL. The therapeutic causing contraction of oral pharyngeal mucosal tissues may be administered all at once, but is preferably injected into a plurality of sites to prevent a rapid change in tissues and treat the tissues more safely, and it is more preferable that the tip of a needle is inserted into submucosa, and while withdrawing the needle, the position of the tip of the needle is changed in the submucosa to inject the therapeutic into a plurality of sites along the prickling pathway. When the therapeutic is administered in divided portions, each portion is preferably 0.01 to 0.5 ml, more preferably 0.05 to 0.3 ml, still more preferably 0.08 to 0.15 ml. Particularly when the ethanol concentration is high, topical denaturation and swelling occur more rapidly, and thus the therapeutic is administeredpreferably into a plurality of site while the injection volume per site is decreased.

When the therapeutic to be administered in the method of treating diseases relating to oral or pharyngeal mucosal tissues according to the present invention does not contain an antihistaminic agent, an antihistaminic agent may be administered before and/or after the administration of the therapeutic. Before injection, mucosal tissues to be treated are preferably subjected to surface anesthesia or infiltration anesthesia with ananesthetic. Basically, anesthesia may be local anesthesia, and general anesthesia is not necessary, thus facilitating treatment. After injection, hospitalization or medical treatment is not necessary, and thus the surgery can be sufficiently finished in one day without hospitalization.

The method of treating various diseases relating to oral or pharyngeal mucosal tissues according to the present invention can contract mucosal tissues while minimizing unspecific inflammatory infiltration and swelling of the mucosal tissues, to ameliorate snoring and obstructive sleep apnea syndrome easily and safely with minimum invasion. Further, the method of treating various diseases relating to oral or pharyngeal mucosal tissues according to the present invention does not involve procedures such as cutting, excision or ablation directly on the surface of the mucosa so that after the treatment, formation of ulcer and scab on the mucosa in the oral cavity and complications of infections in the oral cavity can be minimized, and physical burden on the patient can be reduced. In the method of treating various diseases relating to oral or pharyngeal mucosal tissues according to the present invention, the ethanol-containing therapeutic causing contraction of mucosa is used and is thus extremely inexpensive, highly safe and scarcely causes side effects and aftereffects on humans. In the method of treating various diseases relating to oral or pharyngeal mucosal tissues according to the present invention, an open wound is not formed after administration (injection) of the therapeutic, and the leakage of the injected therapeutic hardly occurs so that the steroid agent is retained very efficiently in the tissues, and its action can be exhibited to the maximum degree, and the content of the additive can be further reduced. When the therapeutic contains additives such as the antihistaminic agent, anesthetic and vasoconstrictor, their action can be exhibited to the maximum degree as well, and the content of the additives can be further reduced.

The side successive pushing injector of the present invention described below is preferably used in the injection of the therapeutic causing contraction of nasal mucosal tissues or the therapeutic causing contraction of oral pharyngealmucosal tissues according to the present invention. That is, a conventionally used usual injector can be used, but unless conducted by skilled persons, there is a high possibility that a large amount of the therapeutic is injected into a site to give severe damage to the mucosal tissues, and thus the side successive pushing injector of the present invention described below is preferably used to reliably prevent injection of the therapeutic all at once in a large amount.

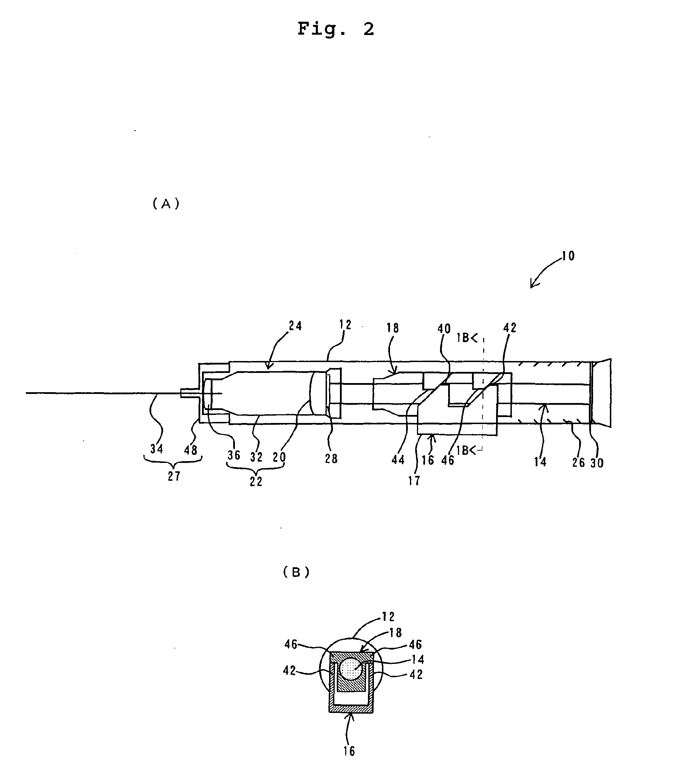

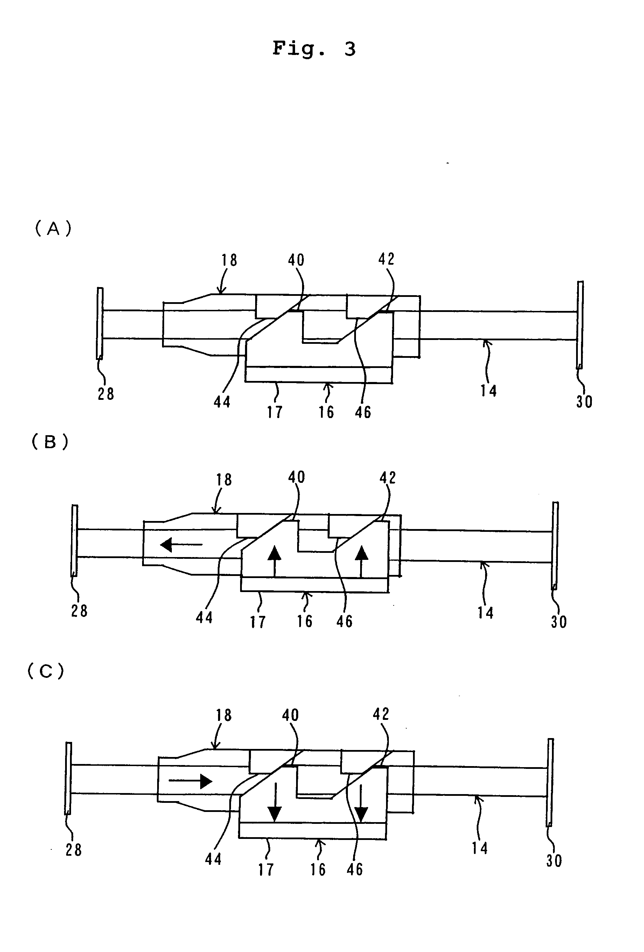

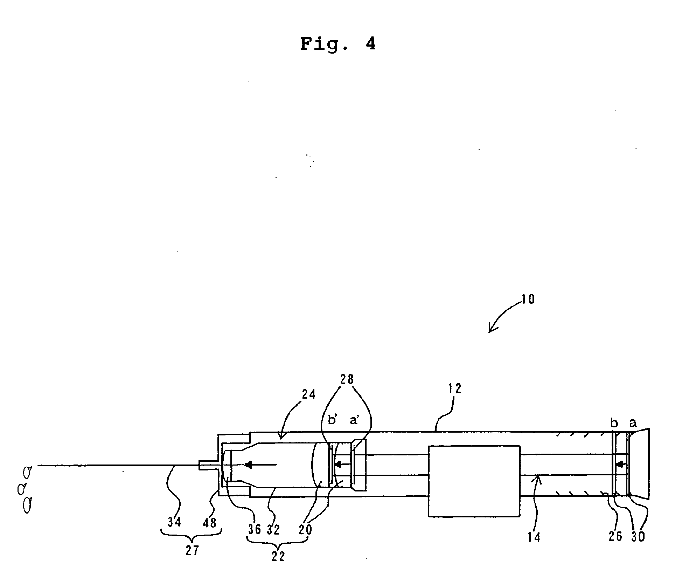

Hereinafter, the injector according to the first embodiment of the present invention is described by reference to the drawings. Fig. 2(A) is a schematic illustration of an injector according to the first embodiment of the present invention; Fig. 2(B) is a sectional view along 1B-1B of the injector shown in Fig. 2(A); Fig. 3 is a set of illustrations showing the movement of a piston in the injector shown in Fig. 2; and Fig. 4 is an illustration of the mechanism of injection of a therapeutic by push of the piston in the injector shown in Fig. 2.

As shown in Fig. 2, the injector 10 according to the first embodiment of the present invention has an outer cylinder 12, a piston 14 capable of moving in the outer cylinder 12, a side projection member 16 arranged to vigorously project from the lateral wall of the outer cylinder 12 to the outside, a switching mechanism 18 engaging with the piston 14 and the side projection member 16, to advance the piston 14 by pressing the side extrusion member 16 toward the axis of the outer cylinder 12, a chemical container accommodating part 24 arranged in the outer cylinder 12 at the advancing side of the piston 14, to accommodate and retain a chemical container 22 equipped with a bottom member 20 capable of being pushed in a watertight state by advance of piston 14, a stopper member 26 (stopper mechanism) preventing return of the advanced piston 14, and a needle member 27 arranged in the top of the outer cylinder 12 and equipped with an injection needle 34.

The outer cylinder 12 is for example a cylinder having an inner diameter of about 10 to 50 mm and a length of about 50 to 200 mm, and is provided therein with piston 14 capable of moving along the axis. The piston 14 is a bar-shaped member having a first disk member 28 in the front end and a second disk member 30 in the rear end, and the first disk member 28 has a diameter slightly smaller than the inner diameter of the chemical container 22, and upon fitting the chemical container 22 into the injector 10, its front side abuts the outside (rear side) of the bottom member 20 sliding in the chemical container 22, and the second disk member 30 has a diameter which is almost the same as the inner diameter of the outer cylinder 12, and the outer periphery thereof contacts the internal surface of the outer cylinder 12.

The piston 14 is provided in the front thereof with a chemical container accommodating part 24, and a chemical container 22 is accommodated in the chemical container accommodating part 24. The chemical container 22 to be accommodated is constituted by tightly closing both ends of a cylindrical body 32 by a stopper member 36 capable of passing the injection needle 34 of the needle member 27 therethrough and a bottom member 20 slidable upon push of the first disk member 28 of piston 14, to enable accommodation of a therapeutic therein. Such chemical container 22 can be constituted so as to accommodate a therapeutic in a predetermined amount such as 0.1 ml, 0.2 ml, 0.3 ml, 0.5 ml or 1.0 ml according to the intended use, etc., and when the injector is used in treatment of diseases relating to the mucosal tissues, the chemical container is constituted to accommodate preferably 0.05 to 2 ml therapeutic, more preferably 0.1 to 1.0 ml therapeutic. The chemical container 22 having a predetermined amount of a therapeutic accommodated therein is thus detachably constituted, so that when a therapeutic containing a volatile substance such as ethanol is to be administered the volatilization of the therapeutic can be prevented. The chemical container accommodating part 24 is preferably provided with e.g. a temperature control means to permit the therapeutic in the chemical container 22 to be kept at a predetermined temperature.

The side projection member 16 is arranged so as to vigorously project by an elastic member from the side of the middle of the outer cylinder 12 to the outside, and can move vertically (vertical direction over Fig. 2) in the lengthwise direction of the piston 14. As shown in Fig. 2(B), the side projection member 16 is a U-shaped member covering the semicircle of lateral surface of the switching mechanism 18, and a first convex 40 and a second convex 42 are arranged at a predetermined anteroposterior interval in both sides of the side projection member 16. The first convex 40 and the second convex 42 are inclined such that the surface of the front side is broadened from upper to lower parts of the convex, and the surface of the rear side extends vertically from upper to lower parts of the convex.

The switching mechanism 18 is a hollow prism with piston 14 penetrating therethrough. Both sides of the switching mechanism 18 are provided with a third convex 44 and fourth convex 46 engaging with the first convex 40 and second convex 42 of the side projection member 16, and the third convex 44 and fourth convex 46 are inclined such that the surface of the front side extends vertically from upper to lower parts of the convex while the surface of the rear side is broadened from upper to lower parts of the convex. The switching mechanism 18 has a piston advancing mechanism capable of advancing piston 14 continuously by maintaining the penetrating piston 14 by pressing the outer periphery of the piston.

The piston advancing mechanism is not particularly limited insofar as the piston can be advanced, and examples include a mechanical pencil system (successive knock system) described in, for example, Japanese Laid-Open Patent Application No. 05-050792, Japanese Laid-Open Patent Application No. 06-008688, Japanese Laid-Open Patent Application No. 07-251595, Japanese Laid-Open Patent Application No. 08-034194, Japanese Laid-Open Patent Application No. 2001-018579, Japanese Laid-Open Patent Application No. 2002-166692, Japanese Laid-Open Patent Application No. 2002-234292, Japanese Laid-Open Patent Application No. 2002-370489, Japanese Laid-Open Patent Application No. 2002-362090, Japanese Laid-Open Patent Application No. 2002-362088, Japanese Laid-Open Patent Application No. 2002-362087, Japanese Laid-Open Patent Application No. 2002-362089, Japanese Laid-Open Patent Application No. 2002-347385, Japanese Laid-Open Patent Application No. 2002-347384, Japanese Laid-Open Patent Application No. 2002-326493, Japanese Laid-Open Patent Application No. 2002-321493, Japanese Laid-Open Patent Application No. 2002-321491, Japanese Laid-Open Patent Application No. 2002-301894, Japanese Laid-Open Patent Application No. 2002-192881, Japanese Laid-Open Patent Application No. 2002-127674, Japanese Laid-Open Patent Application No. 2001-270283, Japanese Laid-Open Patent Application No. 2001-260588, Japanese Laid-Open Patent Application No. 2000-280683, Japanese Laid-Open Patent Application No. 2000-211286, Japanese Laid-Open Patent Application No. 2000-108579, Japanese Laid-Open Patent Application No. 2000-037987, Japanese Laid-Open Patent Application No. 2000-025384, Japanese Laid-Open Patent Application No. 2000-015987, Japanese Laid-Open Patent Application No. 2000-001088 and Japanese Laid-Open Patent Application No. 11-309983.

The inner periphery at the rear of the outer cylinder 12 is provided with a stopper member 26 preventing return of piston 14 having advanced by engaging with the second disk member 30 in piston 14. The stopper member 26 is a plurality of opposing leaves inclined forwards and successively arranged at intervals each corresponding to one advancing distance of piston 14 in the inner periphery at the rear of the outer cylinder 12, to enable the forward movement of piston 14 and secure prevention of the backward movement of piston 14 after piston 14 advances at a predetermined distance (after going beyond one leaf). When the mechanical pencil system is used, the leaves may not necessarilybe arrangedbecause the stopper mechanism is arranged in the system, but the leaves are preferably arranged for more reliably preventing the backward movement of the piston.

Further, the top of the outer cylinder 12 is provided with a needle member 27. The needle member 27 has a fitting part 48 attached detachably to the top of the outer cylinder 12 and an injection needle 34 fixed in the fitting part 48, and when the chemical container 22 is accommodated in the chemical container accommodating part 24 and the needle member 27 is fit thereto, the rear of the injection needle 34 penetrates through the stopper member 36 of the chemical container 22 and is positioned in the chemical container 22.

The injector 10 of the present invention is constituted as illustrated above, and its movement is described in more detail bellow.

In the injector 10 as shown in Fig. 3(A) to (C) and Fig. 4, the press face 17 of the side projection member 16 is pressed to move the side projection member 16 toward the inner direction (vertical upper position over Fig. 2) of the outer cylinder 12, whereby the slanted face in the front side of the first convex 40 in the side projection member 16 and the slanted face in the rear side of the third convex 44 in the switching mechanism 18, and the slanted face in the front side of the second convex 42 in the side projection member 16 and the slanted face in the rear side of the fourth convex 46 in the switching mechanism 18, are allowed to slide, contacted with each other and move such that the switching mechanism 18 moves forwards in a predetermined distance. Simultaneously, the switching mechanism 18 presses and retains the outer periphery of piston 14 penetrated therein by the piston advancing mechanism, to allow the piston 14 to advance (Fig. 3(A), (B)). When the side projection member 16 is fully pressed and the advance of the switching mechanism 18 is finished, the press of the outer periphery of the piston 14 penetrating therein is released (in a non-pressed state), and while the piston 14 is left in the advanced position, the switching mechanism 18 moves backwards to return the original position (Fig. 3(C)). As the piston 14 advances, the second disk member 30 goes beyond one forward stopper member 26 (see Fig. 4), and thus the stopper material 26 reliably prevents piston 14 from moving backwards together with the switching mechanism 18. In the above moving, the piston 14 advances by a predetermined distance from a to b in Fig. 4, so that by the piston 14, the bottom member 20 of the chemical container 22 is pushed forwards by a predetermined distance from a' to b' in Fig. 4, and a predetermined amount of a therapeutic can be injected through the injection needle 34. Then, this movement can be repeatedly carried out to inject the same amount of a therapeutic successively.

In the injector 10 as described above, the advancing distance of the piston by once pressing the side projection member to the end is always constant so that a desired amount of the therapeutic can be jetted. The injector is constituted such that the side projection member projecting from the side is pushed thus reliably preventing slippage in the advancing direction of the injection needle upon pressing, to maintain the injector stably to inject the therapeutic. That is, the therapeutic can be injected accurately and reliably into a suitable site of lesions, and unexpected leakage and scattering of the therapeutic upon injection can be prevented. Particularly, when the therapeutic causing contraction of nasal mucosal tissues or the therapeutic causing contraction of oral pharyngeal tissues is used as a therapeutic, the therapeutic can be injected into suitable sites even when it is to be injected into a plurality of sites in a pricking pathway while the injection needle is withdrawn, and leakage and scattering of the therapeutic can be prevented, and damage to healthy mucosal epithelial tissues on the surface of non-affected mucosa can be prevented.

Further, the injector 10 of the present invention can inject the same amount of the therapeutic as previously by successively pressing the side projection member, and is thus particularly useful when the therapeutic is injected into a plurality of sites in one treatment or injected several times into the same site. For example, after the right nasal mucosa is treated, the needle member is exchanged and the remaining therapeutic can then be injected into the left nasal mucosa, or while the mucosa is prickled with the injection needle, the depth and direction of the tip of the injection needle are changed to inject the therapeutic several times, whereby the therapeutic can be injected into a broad area of submucosa by only one injection, in other words, with minimum invasion.

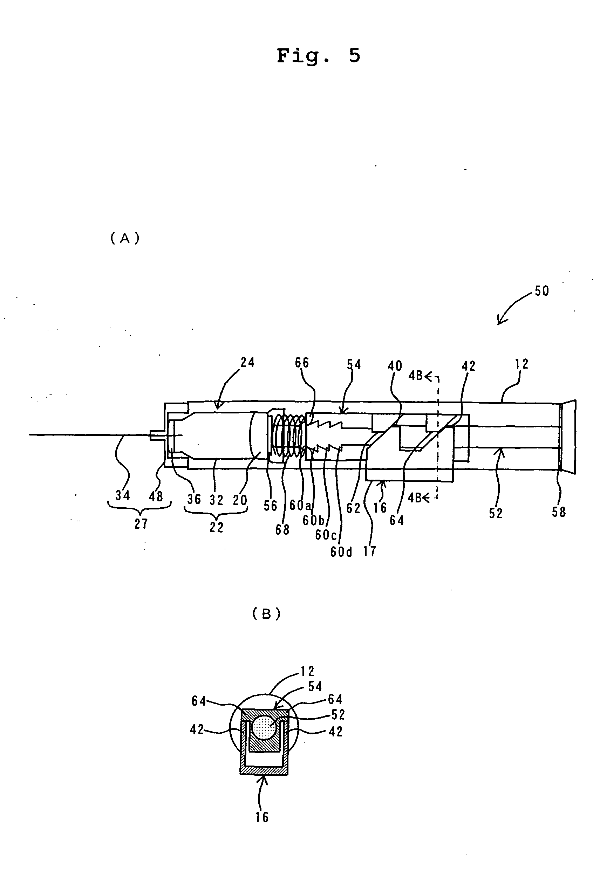

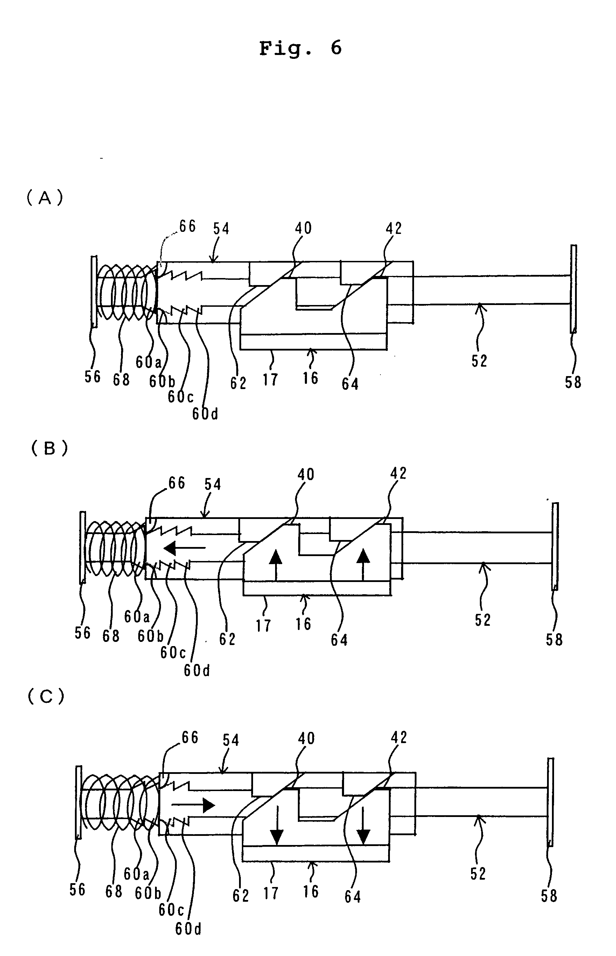

Then, the injector according to the second embodiment of the present invention is described. The same constitution as in the injector 10 according to the first embodiment is provided with the same symbol, and the description is omitted. Fig. 5(A) is a schematic illustration of the injector according to the second embodiment of the present invention, Fig. 5(B) is a sectional view along 4B-4B of the injector shown in Fig. 5(A), and Fig. 6 is a set of illustrations showing the movement of a piston in the injector shown in Fig. 5.

As shown in Fig. 5, the injector 50 according to the second embodiment of the present invention has an outer cylinder 12, a piston 52 capable of moving in the outer cylinder 12, a side projection member 16 arranged to vigorously project from the side of the outer cylinder 12 to the outside, a switching mechanism 54 engaging with the piston 52 and the side projection member 16, to advance the piston 52 by pressing the side projection member 16 toward the inside of the outer cylinder 12, a chemical container accommodating part 24 arranged in the outer cylinder 12 at the advancing side of the piston 52, to accommodate and retain a chemical container 22 equipped with a bottom member 20 capable of being pushed in a watertight state by advance of piston 52, a fixingmechanism as one example of a stoppermechanism preventing return of the advanced piston 52, and a needle member 27 arranged in the top of the outer cylinder 12 and equipped with an injection needle.

The piston 52 is a bar-shaped member having a first disk member 56 in the front end and a second disk member 58 in the rear end, and the first disk member 56 has a diameter slightly smaller than the inner diameter of the chemical container 22, and upon fitting the chemical container 22 into the injector 50, its front side abuts the outside of the bottom member 20 sliding in the chemical container 22, and the second disk member 58 has a diameter which is almost the same as the inner diameter of the outer cylinder 12, and the outer periphery thereof contacts the internal surface of the outer cylinder 12. The piston 52 is provided in the front side of the middle thereof with a plurality of tapered members 60a, 60b, 60c and 60d having the same shape extending backwards. The front of the first disk member 56 of the piston 52 has a fixing mechanism and can fix itself in the outside (rear) of the bottom member 20 in the chemical container 22.

The switching mechanism 54 is a hollow prism with piston 52 penetrating therethrough. The side of the switching mechanism 54 is provided with a third convex 62 and fourth convex 64 engaging with the first convex 40 and second convex 42 of the side projection member 16, and the third convex 6 2 and fourth convex 64 are inclined such that the surface of the front side extends vertically from upper to lower parts of the convex while the surface of the rear side is broadened from upper to lower parts of the convex. Further, the inner periphery of the top in the front of the switching mechanism 54 is provided with a piston pushing member 66 forced to move inside by an elastic member, and before use, its front abuts the rear of the tapered member 60a. Between the first disk member 56 of the piston 52 and the piston pushing member 66, a coil spring 68 as one example of the elastic member is arranged to accommodate piston 52 therein.

The injector 50 of the present invention is constituted as described above, and the movement is described in detail below.

In the injector 50 as shown in Fig. 6(A) to (C), the press face 17 of the side projection member 16 in the injector 50 is pressed to move the side projection member 16 toward the inner direction (vertical upper position over Fig. 5) of the outer cylinder 12, whereby the slanted face in the front side of the first convex 40 in the side projection member 16 and the slanted face in the rear side of the third convex 62 in the switching mechanism 54, and the slanted face in the front side of the second convex 42 in the side projection member 16 and the slanted face in the rear side of the fourth convex 64 in the switching mechanism 54, are allowed to slide, contacted with each other and move such that the switching mechanism 54 moves forwards in a predetermined distance. Simultaneously, the piston pushing member 66 in the switching mechanism 54 presses the rear face of the tapered member 60a, to allow the piston 52 to advance in a predetermined distance (Fig. 6(A), (B)). When the side projection member 16 is fully pressed and the advance of the switching mechanism 54 is finished, the switching mechanism 54 moves backwards by coil spring 68, and the pushing member 66 forced to move inside is extended outside by force by the side of one backward pushing tapered member 60b, to go beyond the tapered member 60b and is simultaneously forced to move inside to abut the rear of the tapered member 60b. The front of the first disk member 56 and the outer side of the bottom member 20 of the chemical container 22 are fixed by the fixing mechanism, so that the piston 52 remains in the advanced position without moving backwards. In the above movement, a predetermined amount of a therapeutic can be jetted through the injection needle 34 (see Fig. 5). Then, this movement can be repeatedly carried out to inject the same amount of a therapeutic successively.

In the injector 50 as described above, the advancing distance of the piston by once pressing the side projection member to the end is always constant so that a desired amount of the therapeutic can be jetted. The injector is constituted such that the side projection member projecting from the side is pushed thus reliably preventing slippage in the advancing direction of the injection needle upon pressing, to maintain the injector stably to inject the therapeutic. That is, the therapeutic can be injected accurately and reliably into a suitable site of lesions, and unexpected leakage and scattering of the therapeutic upon injection can be prevented. Particularly, when the therapeutic causing contraction of nasal mucosal tissues or the therapeutic causing contraction of oral pharyngeal mucosal tissues is used as a therapeutic, the therapeutic can be injected into suitable sites even when it is to be injected into a plurality of sites in a pricking pathway while the injection needle is withdrawn, and leakage and scattering of the therapeutic can be prevented, and damage to healthy mucosal epithelial tissues on the surface of non-affected mucosa can be prevented.

Further, the injector 50 of the present invention can inject the same amount of the therapeutic as previously by successively pressing the side projection member, and is thus particularly useful when the therapeutic is injected into a plurality of sites in one treatment or injected several times into the same site. For example, after the right nasal mucosa is treated, the needle member is exchanged and the remaining therapeutic can then be injected into the left nasal mucosa, or while the mucosa is stuck with the injection needle, the depth and direction of the tip of the injection needle are changed to inject the therapeutic several times, whereby the therapeutic can be injected into a broad area of submucosa by only one injection, in other word, with minimum invasion.

Then, the injector according to the third embodiment of the present invention is described. The injector according to the third embodiment is useful in administering two kinds of chemicals. Conventional injectors for administering two kinds of chemicals include those described in Japanese Laid-Open Patent Application No. 7-136267, Japanese Laid-Open Patent Application No. 7-148261, Japanese Laid-Open Patent Application No. 7-136264, Japanese Laid-Open Patent Application No. 6-142203, Japanese Laid-Open Patent Application No. 6-181985, Japanese Laid-Open Utility Model Application No. 5-152, Japanese Laid-Open Patent Application No. 4-246364, Japanese Laid-Open Patent Application No. 62-14863, Japanese Laid-Open Patent Application No. 62-5357, Japanese Laid-Open Patent Application No. 64-80371, Japanese Laid-Open Patent Application No. 7-299141, Japanese Laid-Open Patent Application No. 7-265423, Japanese Laid-Open Patent Application No. 9-308688 and Japanese Laid-Open Patent Application No. 51-11691, but these have complicated structures and are different in concept in the injector of the present invention capable of successive injection.

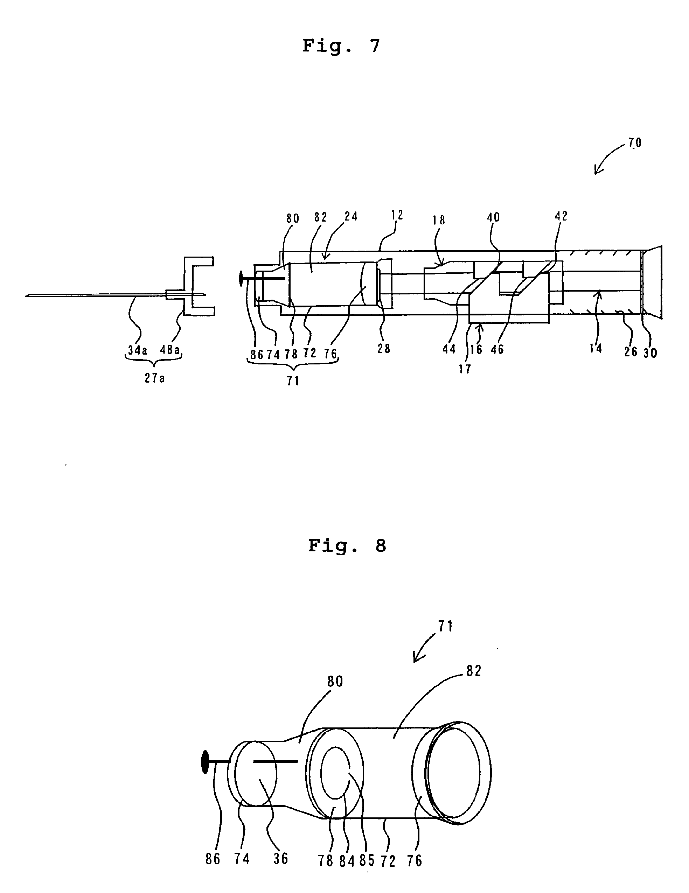

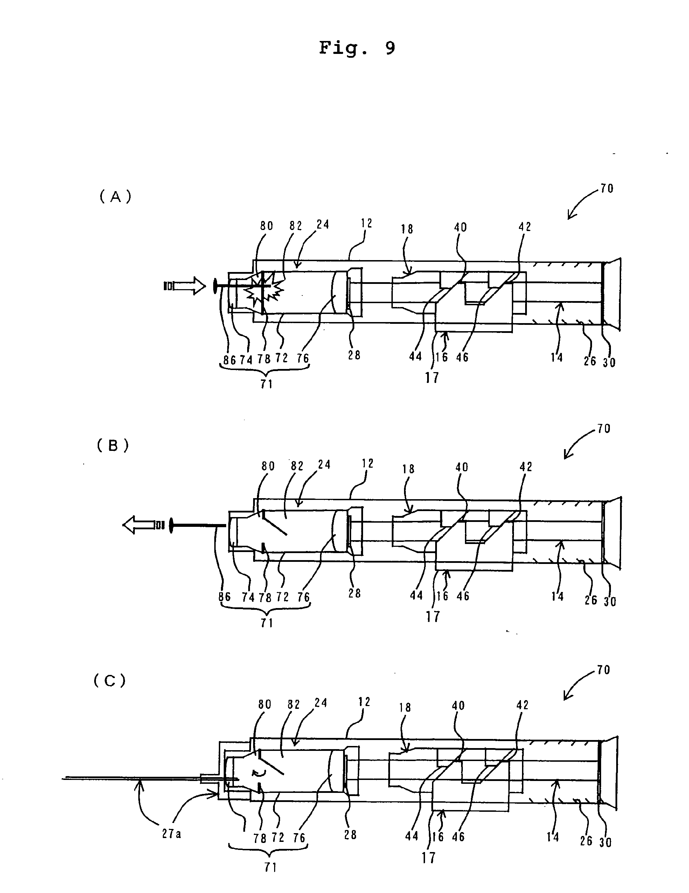

Now, the injector according to the third embodiment of the present invention is described by reference to the drawings. The same constitution as in the injector 10 according to the first embodiment is provided with the same symbol and the description is omitted. Fig. 7 is a schematic illustration of the injector according to the third embodiment of the present invention, Fig. 8 is a perspective view of a chemical container accommodated in the injector shown in Fig. 7, and Fig. 9 (A) to (C) is a drawing showing use of the injector shown in Fig. 7.

As shown in Figs. 7 and 8, the injector 70 according to the third embodiment accommodates a chemical container 71 in a chemical container accommodating part 24. In the chemical container 71 accommodated in the chemical container accommodating part 24, both sides of the cylindrical body 72 (body part) is closed with a stopper member 74 capable of passing an injection needle 34a of the injection needle member 27a therethrough and a bottom member 76 capable of sliding by pushing of a first disk member 28 of piston 14, thus allowing a therapeutic to be accommodated therein. In the vicinity of the stopper member 74 of the cylindrical body 72, a partition wall 78 separating chemicals is arranged to form a chemical chamber 80 for accommodating chemical A and a chemical chamber 82 for accommodating chemical B, and the chemical chamber 80 accommodates a chemical of lower proportion and the chemical chamber 82 accommodates a chemical of higher proportion. The chemicals A and B are not particularly limited, and when they are used in treatment of various diseases relating to mucosal tissues, the chemical A includes, for example, a steroid agent, an antihistaminic agent etc., and the chemical B includes a therapeutic containing ethanol as the active ingredient. The peripheral region of the partition wall 78 is fixed to the inside of the cylindrical body 72, and a break induction part 84 (see Fig. 8) is formed on the surface and/backside of the partition wall 78. The break induction part 84 is a C-shaped linear part formed in the center of the partition wall 78 and is thinner than the other part of the partition wall 78, and the partition wall 78 is easily broken along the break induction part. The break induction part 84 of C-shape as described above forms a linking part 85, thus preventing a situation wherein a part of the partition wall 78 cut off from the body of the partition wall prevents the injection of the therapeutic through the injection needle, or the sliding of the bottom member 76.

The chemical container 71 is provided with a pin member 86 penetrating through a stopper member 36, and the top of the pin member is positioned in the chemical chamber 80, and the head of the pin member 86 is pushed towards the inside of the chemical container 71, whereby the tip of the pin member 86 is pressed against the partition wall 78 to break the partition wall 78 along the break induction part 84. The pin member 86 is arranged such that the tip of the pin member 86 is positioned in the vicinity of the linking region 85 inside the C-shaped break induction part 84, and the partition wall is broken enough along the break induction part 84 to form a large opening so that two kinds of chemicals can be sufficiently mixed with each other. As shown above, the partition wall 78 can be arranged in the vicinity of the stopper member 74, to break the partition wall 78 easily by the pin member 86.

The injection needle member 27a (see Fig. 7) attached to the top of the injector 70 has a fitting part 48a attached detachably to the top of the outer cylinder 12 and an injection needle 34a fixed to a fitting part 48a. When the injection needle 34a penetrates through the stopper member 74 of the chemical container 71, the injection needle 34a is excentrically fixed to the fitting part 48a so as not to prevent emission of the therapeutic from the injection needle 34a by contacting with a part of the partition wall 78 broken at the break induction part 84 (see Fig. 7 and Fig. 9 (C)). Further, the partition wall 78 broken by the break induction part 84 is in a movable state so that upon injection, the partition wall 78 is naturally pushed back to the front side of the chemical chamber 80 by sliding of the bottom member 76, and thus the chemical liquid in the chemical container can be utilized without waste (see Fig. 9 (C)).

To use the injector 70 constituted as described above, the pin member 86 is first pushed to break the partition wall 78 along the break induction part 84 (see Fig. 8), as shown in Fig. 9 (A). The respective chemicals accommodated respectively in the chemical containers 80 and 82 can be safely, easily and rapidly mixed with each other. As shown in Fig. 9 (B), the pin member 86 is then pulled off, and as shown in Fig. 9 (C), the injection needle member 27a is then fitted to it. Then, the side projection member 16 of the injector 70 is pushed in the same manner as in the injector 10, whereby the mixed therapeutic can be injected in the same amount accurately and successively.

In addition to the same effect as that of the injectors 10 and 50, the injector 70 uses the above chemical containers by which the respective chemicals are separated completely from each other just until performing injection, and thus unnecessary mixing of the respective chemicals is prevented during storage until injection, and a difference in injection effect attributable to the change in qualities of the respective chemicals caused easily during storage of a composition of plural medicines such as a blended preparation can be prevented, and the safety of the blended preparation in the living body can also be improved. That is, problems such as quality control and storage of the blended preparation prior to injection may practically not be taken into consideration, and the therapeutic canbeinjectedmoresafely. Further, acomplicateddevelopmental process necessary for production of a new blended liquid drug with different kinds of chemicals can be omitted or simplified. Further, problems such as sterilization of chemicals charged into an injector, which has been regarded as difficult, can be solved more easily at the level of production of the chemical container. In addition, the structure of the injector 70 is extremely simple, and complicated techniques are not necessary, thus realizing production of the lower-cost product.

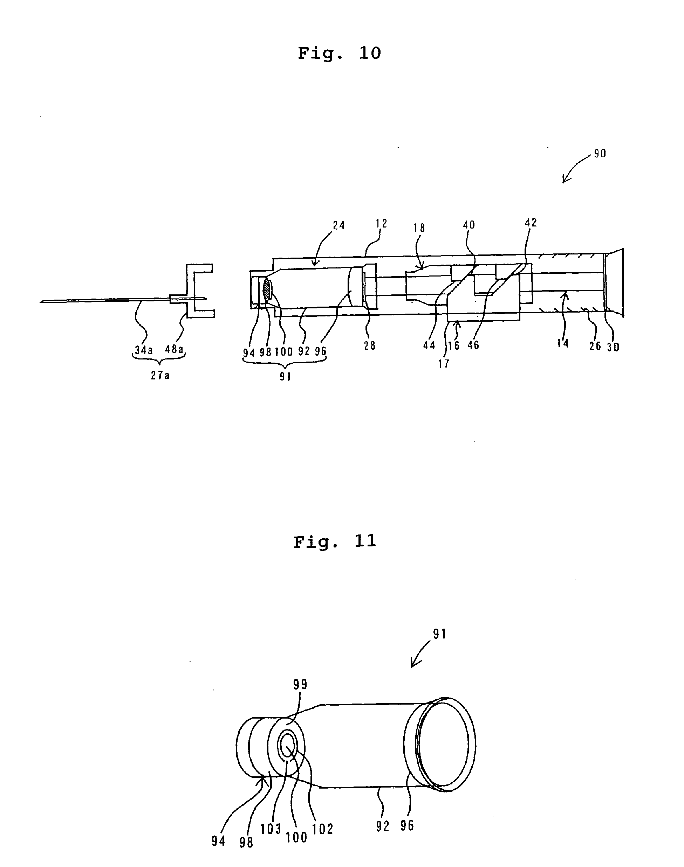

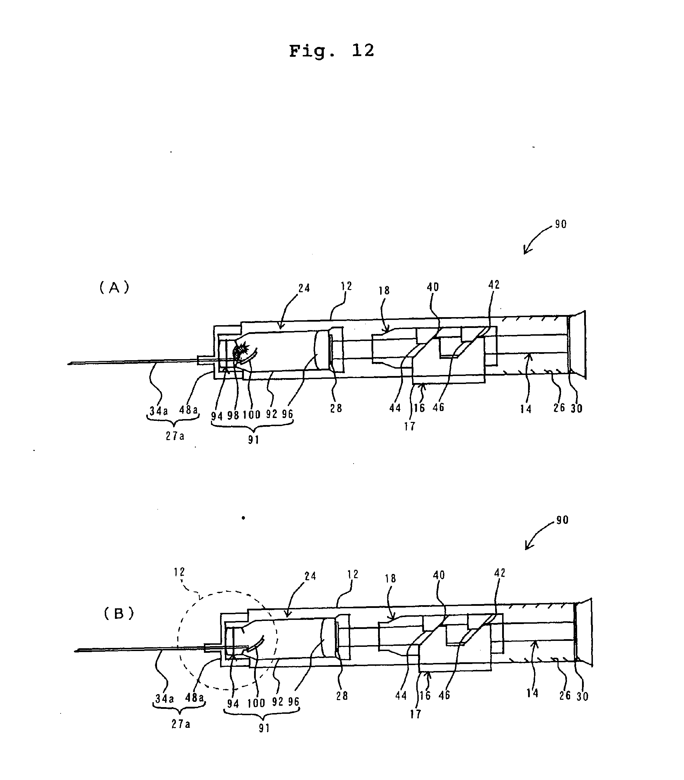

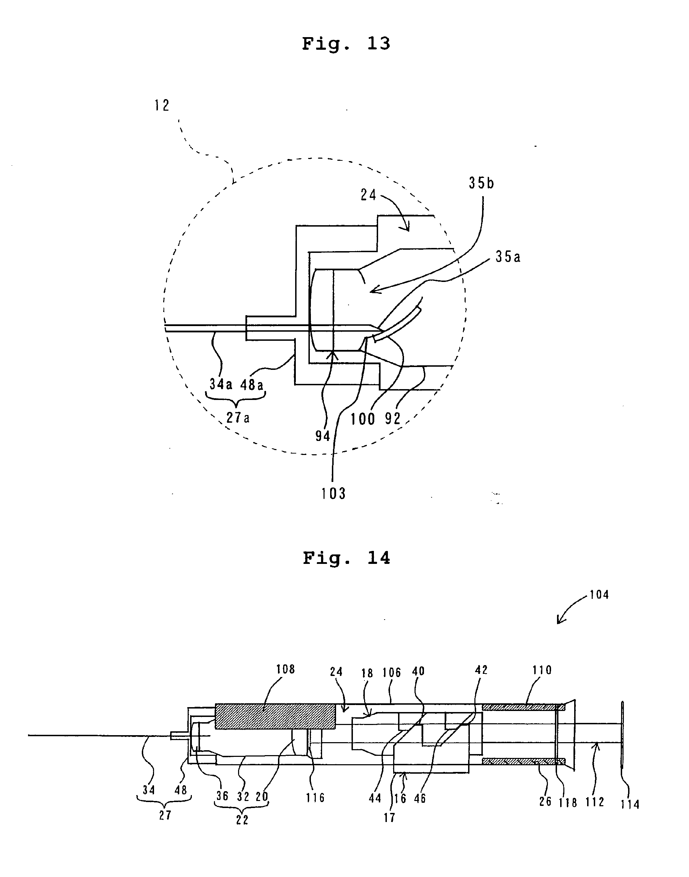

Then, the injector 90 according to the fourth embodiment of the present invention is described. The injector according to the fourth embodiment of the present invention, similar to the injector 70, is useful in administering two kinds of chemicals. The same constitution as in the injector 70 is provided with the same symbol and the description is omitted. Fig. 10 is a schematic illustration of the injector according to the fourth embodiment of the present invention, Fig. 11 is a perspective view of a chemical container accommodated in the injector shown in Fig. 10, Fig. 12(A), (B) is a drawing showing use of the injector shown in Fig. 10, and Fig. 13 is a partially enlarged view of the injector shown in Fig. 12.

As shown in Figs. 10 and 11, the injector 90 according to the fourth embodiment of the present invention accommodates a chemical container 91 in a chemical container accommodating part 24. In the chemical container 91 accommodated in the chemical container accommodating part 24, both sides of a cylindrical body 92 (body part) is closed by a stopper member 94 capable of penetrating the injection needle 34a of the injection needle member 27a therethrough and the bottom member 96 capable of sliding by pushing of the first disk member 28 of piston 14, thus allowing a therapeutic to be accommodated therein. In the stopper member 94, a chemical accommodating chamber 98 is arranged, and for example, a steroid agent, an antihistaminic agent etc. are accommodated therein. The outer face 99 in the bottom of the chemical accommodating chamber 98 is provided with a break induction part 102, and a disk-shaped supporting member 100 is fixed in the position a little closer to the center (inside). The supporting member 100 is a rigid member for supporting the bottom of the chemical accommodating chamber 98 composed generally of flexible material, to prevent elongation of the material by pressing and to facilitate breakage of the bottom of the chemical accommodating chamber 98. The break induction part 102 is a C-shaped linear part surrounding the supporting member 100, and is thinner than the other part of the bottom of the chemical accommodating chamber 98 so that by attachment of the injection needle member 27a and simultaneously pressing the bottom of the chemical accommodating chamber 98 at the rear end of the injection needle 34a, the bottom of the chemical accommodating chamber 98 is broken along the break induction part 102. The break induction part 102 of C-shape as described above forms a linking part 103 (see Fig. 11), thus preventing a situation wherein a part of the bottom of the chemical accommodating chamber 98 cut off from the main body of the bottom of the chemical accommodating chamber 98 prevents the injection of the therapeutic through the injection needle, or the sliding of the bottom member 96.

The injection needle member 27a (see Fig. 10) attached to the top of the injector 90 has a fitting part 48a attached detachably to the top of the outer cylinder 12 and an injection needle 34a fixed to the fitting part 48a. The injection needle 34a in the injection needle member 27a penetrates through the stopper member 94 of the chemical container 91 and simultaneously breaks the bottom of the chemical accommodating chamber 98, and the injection needle 34a is fixed with eccentricity to the fitting part 48a (see Fig. 13) such that the injection needle 34a can push and open a part in the vicinity of the linking part 103 inside the C-shaped break induction part 102 in the bottom of the chemical accommodating chamber 98 (see Fig. 11), to form a sufficiently large opening along the break induction part 102 (see Fig. 11) to permit two kinds of chemicals to be sufficiently mixed with each other. Preferably a mark is given to the outer cylinder 12, the chemical container 91 and the injection needle member 27a so as to enable constant fitting in the direction along circumference of the chemical container 91 and the injection needle member 27a. As shown in Fig. 13, the injection needle 34a has a structure wherein the bent state of the linking part 103 is maintained by the part of the injection needle 34a penetrating into the chemical accommodating chamber 98, so that the injection of a therapeutic is not disturbed by clogging or covering of opening 35b formed in the supporting member 100 with the portion of the supporting member after opened by the inlet 35a of the injection needle 34a. Simultaneously, the inlet 35a of the injection needle 34a is cut off in slant sharp angle so that the injection fluid can be discharged more smoothly though the injection needle. For example, the inlet 35a of the injection needle 34a is preferably structured to be slanted (sharp angle) so as to be longer at the side of the linking part 103, and a part of the bottom of the chemical accommodating chamber 98 is supported at the part of the sharp angle.

To use the injector 90 constituted as described above, the injection needle member 27a is first fitted to the top of the outer cylinder 12 as shown in Fig. 12(A) and (B), and the bottom of the chemical accommodating chamber 98 is pushed by the rear end of the injection needle 34a, to break the bottom of the partition wall 98 along the break induction part 102 (see Fig. 11). Chemicals accommodated respectively in the chemical accommodating chamber 98 and chemical containers 91 can be safely, easily and rapidly mixed with each other. Then, the side projection member 16 of the injector 90 is pushed in the same manner as in the injector 10, whereby a very small amount of the mixed therapeutic can be injected in the same amount accurately and successively.

In addition to the same effect as that of the injectors 10 and 50, the injector 90 uses the above chemical containers by which the respective chemicals are separated completely from each other just until injection, and thus unnecessary mixing of the respective chemicals is prevented during storage until injection, and a difference in injection effect attributable to the change in qualities of the respective chemicals caused easily during storage of a composition of plural medicines under a blended condition can be prevented, and the safety of the blended preparation in the living body can also be improved. That is, problems such as quality control and storage of the blended preparation prior to injection may practically not be taken into consideration, and the therapeutic can be injected more safely. Further, a complicated developmental process for a new blended drug necessary for production of a blended liquid of different kinds of chemicals can be omitted or simplified. Further, problems such as sterilization of chemicals charged into an injector, which has been regarded as difficult, can be solved more easily at the level of production of the chemical container. In addition, the structure of the injector 90 is extremely simple, and complicated techniques are not necessary, thus realizing production of the lower-cost product.

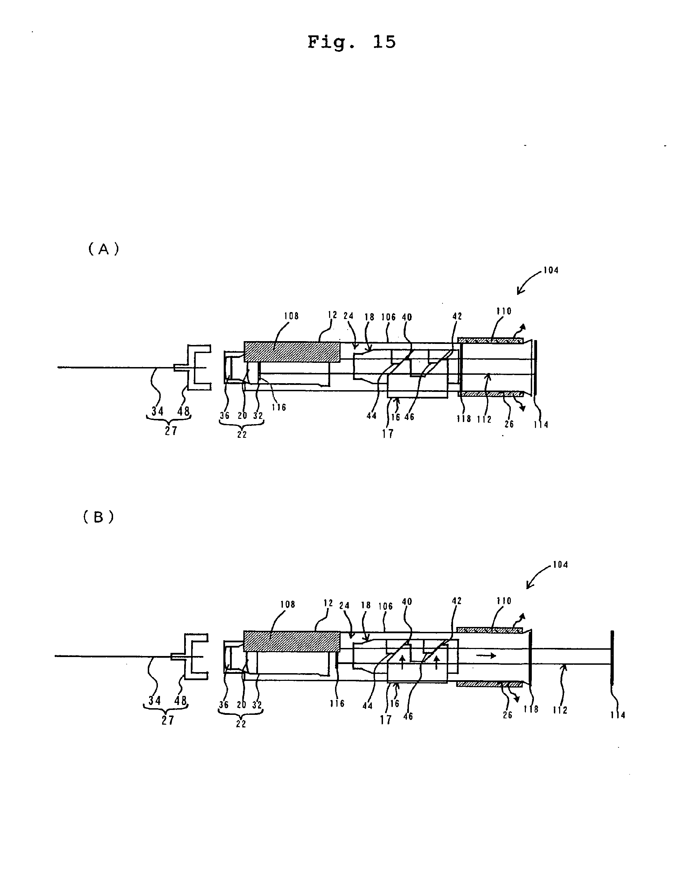

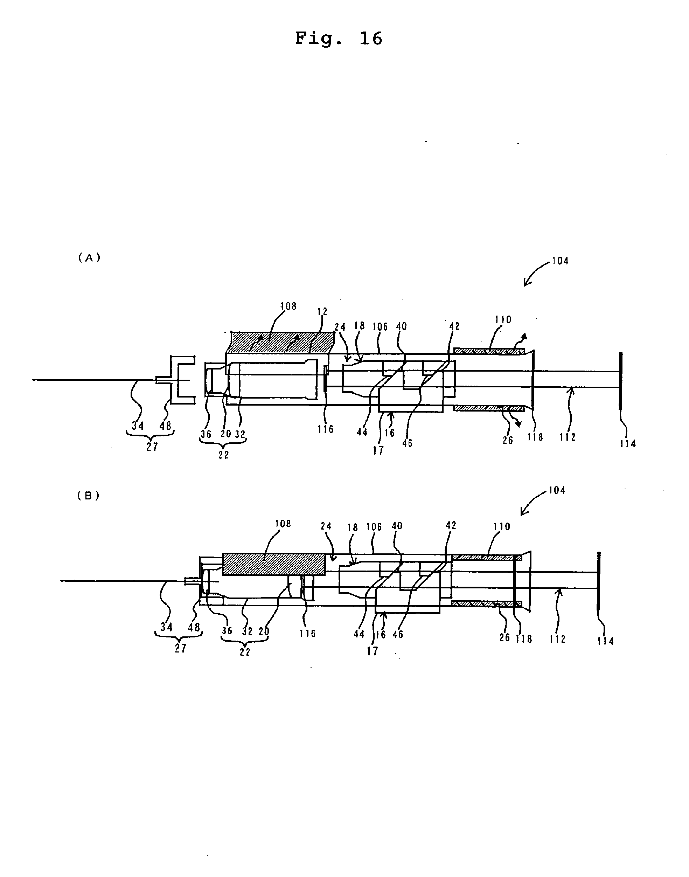

Then, the injector according to the fifth embodiment of the present invention is described. The injector according to the fifth embodiment of the present invention is usable successively by exchanging the chemical container only. The same constitution as in the injector 10 is provided with the same symbol and the description is omitted. Fig. 14 is a schematic illustration of the injector according to the fifth embodiment of the present invention, and Fig. 15(A), (B) and Fig. 16(A), (B) are drawings showing use of the injector shown in Fig. 14.

As shown in Fig. 14, the outer cylinder 106 of the injector 104 according to the fifth embodiment of the present invention has an opening window 108 capable of introducing and removing the chemical container 22 at the position of the side chemical container accommodating part 24 (front side) and an opening window 110 at the position of the stopper member 26. The piston 112 protrudes its end from the rear of the outer cylinder 106 and is provided with a third disk member 114 in the rear thereof (rear end). By arranging the third disk member 114, the third disk member 114 is retained to facilitate backward movement of piston 112. Like the injector 10, the piston 112 has the first disk member 116 and the second disk member 118.

In the method of exchanging the chemical container 22 of the injector 104 having such constitution, as shown in Fig. 15 (A), a therapeutic is jetted out (piston 112 is advanced), and then the injection needle member 27 is removed, and then the opening window 110 is opened, whereby the engagement of the stoppermember 26 with the piston 112 is dissociated. As shown in Fig. 15(B), the side projection member 16 is pressed thereby dissociating the engagement of the switching mechanism 18 with the piston 112, and the third disk member 114 is retained to move the piston 112 backwards so that the first disk member 116 of the piston 112 is in such a state as not to contact with the chemical container 22. As shown in Fig. 16 (A), the opening window 108 is then opened, and the used chemical container 22 is removed, and a new chemical container is introduced. As shown in Fig. 16(B), the side projection member 16 is finally pressed to allow the first disk member of the piston 112 to abut the bottom member 20 of the chemical container 22, to close the opening windows 108, 110, and a new injection needle member is fitted to enable subsequent use. By thus arranging the opening windows 108, 110, the exchange of the chemical container 22 can be conducted easily and smoothly. Then, the above procedure is repeated, whereby the injector 104 can be repeatedly used. In this embodiment, the chemical container 22 is used, but the chemical container 71 or 91 can also be naturally used.

In addition to the same effect as that of the injectors 10 and 50, the injector 104 can be reutilized many times until it is broken, and thus the medical waste generated after each injection is only the chemical container, and as compared with disposal injectors, the medical waste can be significantly reduced. From the viewpoint of production , the chemical container only is produced in a large amount and supplied, and thus an energy-saving product with minimum waste can be developed.

The injector of the present invention has been described in detail by reference to some embodiments, but the present invention is not limited thereto; for example, a part or the whole of an injection needle of the needle member can be curved, and in this case, the direction of the top of the needle relative to the axial direction of the injection needle (axial direction of the outer cylinder) (that is, the angle between the top of the curved needle and the original position of the top of the needle) is preferably 0° to 130°, more preferably 10° to 90°, still more preferably 20° to 70°. When a part (top) of the injection needle is curved, the length of the curved top of the injection needle is preferably 5 to 40 mm, more preferably 10 to 30 mm. The therapeutic can thereby be injected accurately into sites into which it is hardly injected, such as oral pharyngolaryngeal submucosa and nasal submucosa. Specifically, when the therapeutic is injected into a uvular site, the injection needle should be inserted upward through the top of the uvula, and when the injection needle is curved, the injection needle can be accurately inserted to inject the therapeutic.

The injector can be constituted such that the rear of the piston can be extruded from the rear of the outer cylinder, and in this case, the injector, similar to a usual injector, can be constituted so as to be usable by pressing the piston from the rear. Further, the press face of the side projection member may be provided with a nonslip region by which the therapeutic can be injected more safely.

The injector of the present invention can also be constituted such that the chemical container can be introduced and removed through the front. The shape of the break induction part formed in the partition wall or in the bottom of the chemical accommodating part is not particularly limited. For example, when it is in an arc form, a connecting member connecting the outside of the arc to the inside may be arranged, thus preventing a situation wherein a part of the partition wall or the bottom of the chemical accommodating chamber which has been cut off prevents the injection of the therapeutic through the injection needle, or the sliding of the bottom member. The injector of the present invention can be used to inject not only the therapeutic causing contraction of nasal mucosal tissues and the therapeutic causing contraction of oral pharyngeal tissues but also other chemicals such as insulin. The injector of the present invention may be a disposal injector, or may be an injector which can be used successively by exchanging the chemical container only. The injector may be combined with a chemical container to form a therapeutic set. In this case, it may be a therapeutic set having the chemical container accommodated in the body of the injector or may be a therapeutic set having the injector and the chemical container accommodated in an accommodation casing. In consideration of the shape etc. of the injector and the chemical container such that a very small amount of the therapeutic can be injected several times, the injector is constituted such that preferably 0.01 to 0.5 ml, more preferably 0.03 to 0.3 ml, still more preferably 0.08 to 0.15 ml can be injected by once pressing the side projection member of the injector.

Hereinafter, the present invention is described in more detail with reference to the Examples, but the technical scope of the present invention is not limited to these examples. In the Examples, "%" refers to "% by mass" unless otherwise specified.

### [Example 1-1]

### (Ethanol treatment)

Under the consent of the patient, the following treatment was carried out. The patient was a 27-year-oldwomanmainlyhaving nasal congestion, rhinorrhea and sneezes and diagnosed in clinical diagnosis to have allergic rhinitis of seasonal and perennial mixed type.

In pretreatment, the surface anesthesia of right inferior turbinate mucosa was conducted with 4% xylocaine (with epinephrine hydrochloride added at 1 : 1000). Then, the tip of a curved 23G Cateran injection needle was contacted slightly with the right inferior turbinate bone and simultaneously advanced beyond the middle part of the inferior nasal concha, and the injection needle was withdrawn and simultaneously injected a therapeutic (0.2 ml of 70% ethanol) causing contraction of nasal mucosal tissues into 2 sites of the submucosa in the vicinity of the center of the most swollen mucosa. Thereafter, the injection needle was left in the vicinity of the pricked site without withdrawing the injection needle all at once, and after a dozen of seconds, the injection needle was withdrawn. After the injection was completed, an epinephrine (styptic) gauze was placed and pressed for 10 minutes upon the inferior turbinate mucosa to stop the bleeding, to finish the treatment.

### [Comparative Example 1-1]

### (Control treatment)

The following control treatment was conducted for the same patient as in Example 1-1.

In pretreatment, the surface anesthesia of left inferior turbinate mucosa was conducted for 20 minutes with 4% xylocaine (with epinephrine hydrochloride added at 1 : 1000). Using a high-frequency heating device Coblator (ENTEC Coblation System, manufactured by Earth Locare), the end of an electrode probe was inserted into 2 sites in total of inferior turbinate submucosa, and a high-frequency current (90 W; the temperature of the end of the probe was 65°C) was applied for 10 seconds to each site, to ablate the inferior turbinate mucosa. After ablation, the inferior turbinate mucosa was pressed with an epinephrine (styptic) gauze for 10 minutes to stop the bleeding, and finished the treatment.

### [Evaluation]

Before and after the ethanol treatment and the control treatment (after 1 week, after 2 weeks, after 4 weeks and after 8 weeks), the inferior turbinate mucosal tissues were observed with a fiber scope. Before and after the ethanol treatment and the control treatment (after 2 weeks, after 4 weeks and after 8 weeks), the nasal airway patency (nasal airway resistance) was measured. The nasal airway resistance objectively reflecting the nasal airway patency was determined by an anterior method with a mask (anterior induction method) with a nasal airway patency meter (KOC-8900 manufactured by Chest M I). As the standard nasal airway resistance in normal humans, the nasal airway resistance in one of right and left nasal cavities during breathing at 100 Pascal is 1.2 (Pa/ml/s) or less, and the resistance in both of nasal cavities is 0.3 (Pa/ml/s) or less, and the normal humans are defined as humans who have never suffered from nasal diseases and not particularly conscious of nasal congestion daily (Method 1 for Objective Measurement of Nasal Congestion - Acoustic Rhinometry - Makoto Hasegawa, Nobutaka Kawai, Kenichi Motohashi, Hidekazu Tanaka, Akio Ichikawa, Johns, 16:1547-1551, 2000). Further, the ameliorated state of synthetic nasal conditions (sneezes, rhinorrhea, nasal congestion, hindrance in daily life) of the patient subjected to the ethanol treatment and control treatment was examined on the basis of the evaluation criteria shown in Table 4 below.

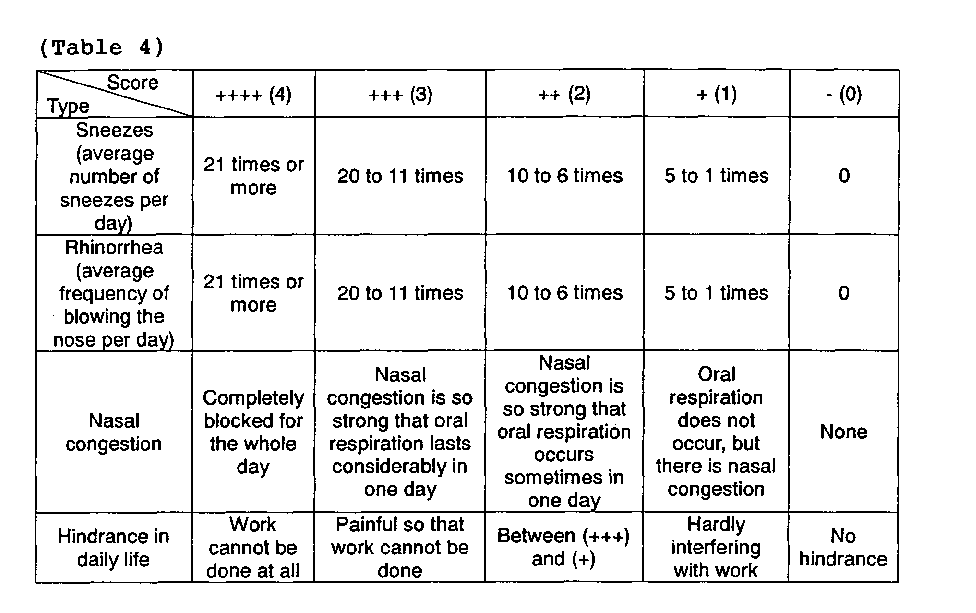

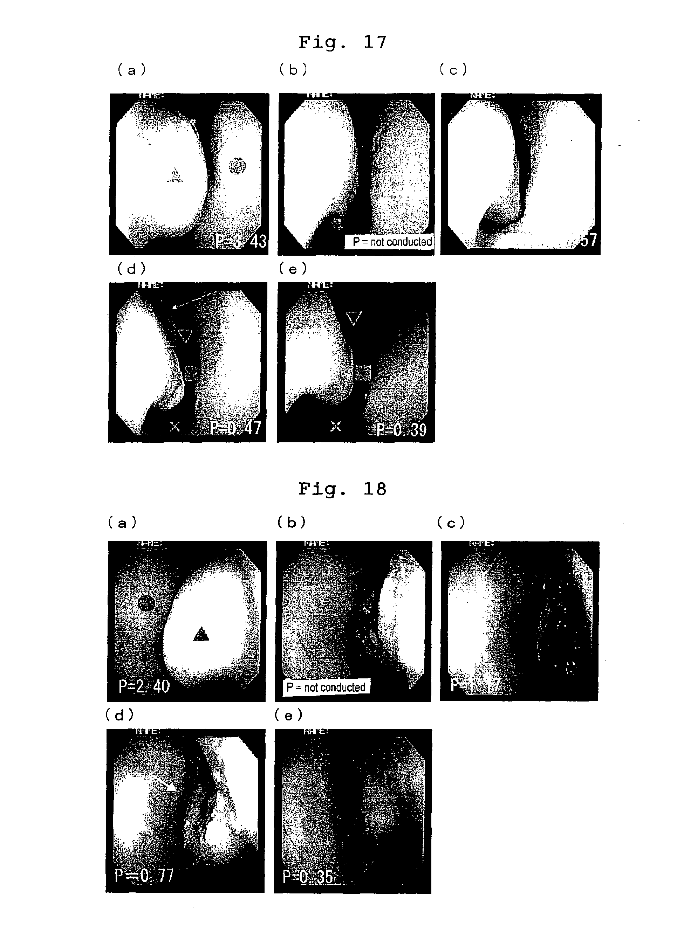

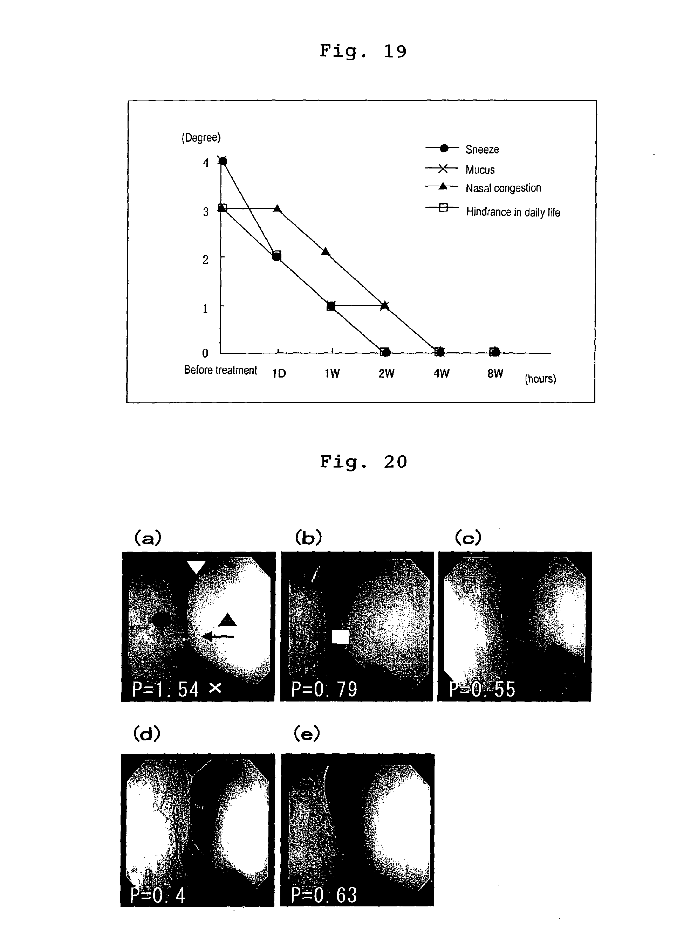

Fig. 17 is a set of photographs showing the observation result of the right inferior turbinate mucosal tissues by a fiber scope before and after the ethanol treatment, wherein (a) is before the treatment, (b) is after 1 week, (c) is after 2 weeks, (d) is after 4 weeks, and (e) is after 8 weeks. In Fig. 17, ● shows the nasal septum, ▲ shows the inferior concha, ∇ shows the middle nasal meatus, ■ shows the common nasal meatus, and × shows the inferior nasal meatus, and the part shown by the arrow indicates the middle nasal concha. Fig. 18 is a set of photographs showing the observation result of the left inferior turbinate mucosal tissues before and after the control treatment, wherein (a) is before the treatment, (b) is after 1 week, (c) is after 2 weeks, (d) is after 4 weeks, and (e) is after 8 weeks. In Fig. 18, ● shows the nasal septum, ▲ shows the inferior concha, ▼ shows the middle nasal meatus, ■ shows the common nasal meatus, and x shows the inferior nasal meatus, and the part shown by the arrow indicates the middle nasal concha. In Figs. 17 and 18, P shows the airway resistance in the left and right nasal cavities (Pa/ml/s) during inspiration at 100 Pascal. Fig. 19 is a graph showing the evaluation result based on the evaluation criteria in Table 4 with time after the ethanol treatment and control treatment of the patient.

### [Results]

As is evident from Fig. 17 (ethanol treatment), after the therapeutic was injected into the inferior turbinate submucosa in the method of treatment with the ethanol-containing therapeutic causing contraction of nasal mucosa according to the present invention, the contraction of the mucosa became significant with time, and opening of each nasal meatus become clear. Particularly, after 4 weeks (see (d), (e)), the middle nasal concha hidden until then could be certainly observed after the opening of the middle nasal meatus. It was also found that with respect to the damage to the mucosal surface after the operation and the degree of formation of scabs, the ethanol treatment was considerably milder than in the control treatment. As is evident from the reliable reduction (reduction of the right nasal airway resistance from 3.43 Pa/ml/s before the treatment to 0.39 Pa/ml/s 8 weeks after the treatment) of the nasal airway resistance after the treatment shown in Fig. 17, a significant effect on opening of the nasal cavity (nasal meatus) was recognized in the therapeutic method using the therapeutic of the present invention.

On the other hand, as shown in Fig. 18, a similar contraction effect to that of the ethanol treatment was obtained in the control treatment, but for not so short while after ablation, a strong ulcerous lesion on the mucosal surface was brought about, and a longer time was required for recovery from the mucosal wound.

As is evident from Fig. 19, the subjective symptoms of the patient subjected to the ethanol treatment and control treatment indicated that nasal symptoms, particularly sneezes and rhinorrhea, were ameliorated immediately from the next day, and the feeling of nasal congestion was also cancelled after 4 weeks. Further, use of an oral medicine for nasal allergy was not necessary after 2 weeks, and the hindrance in daily life became 0. That is, although the high-frequency tissue reducing method conducted conventionally for other diseases was applied to the left nasal cavity, a certain therapeutic effect on the nasal symptoms was obtained as a whole, and thus it can be said that a significant therapeutic effect on nasal symptoms is evidently obtained by the therapeutic method of the present invention.

### [Example 1-2]

Under the consent of the patient, the following treatment was carried out. The patient was a 20-year-old woman mainly having nasal congestion, rhinorrhea and sneezes and diagnosed in clinical diagnosis to have allergic rhinitis of seasonal and perennial mixed type.

Both the nasal cavities of the patient were treated in the same manner as in Example 1-1. As the therapeutic, 70% ethanol was used, and 0.3 ml was injected into each nasal cavity.

### [Evaluation]

Before and after the treatment (after 2 weeks, after 4 weeks, after 8 weeks and after 16 weeks), the inferior turbinate mucosal tissues were observed with a fiber scope. The ameliorated state of the synthetic nasal conditions (sneezes, rhinorrhea, nasal congestion, hindrance in daily life) of the patient subjected to the treatment in Example 1-2 was examined on the basis of the evaluation criteria shown in Table 4 above.

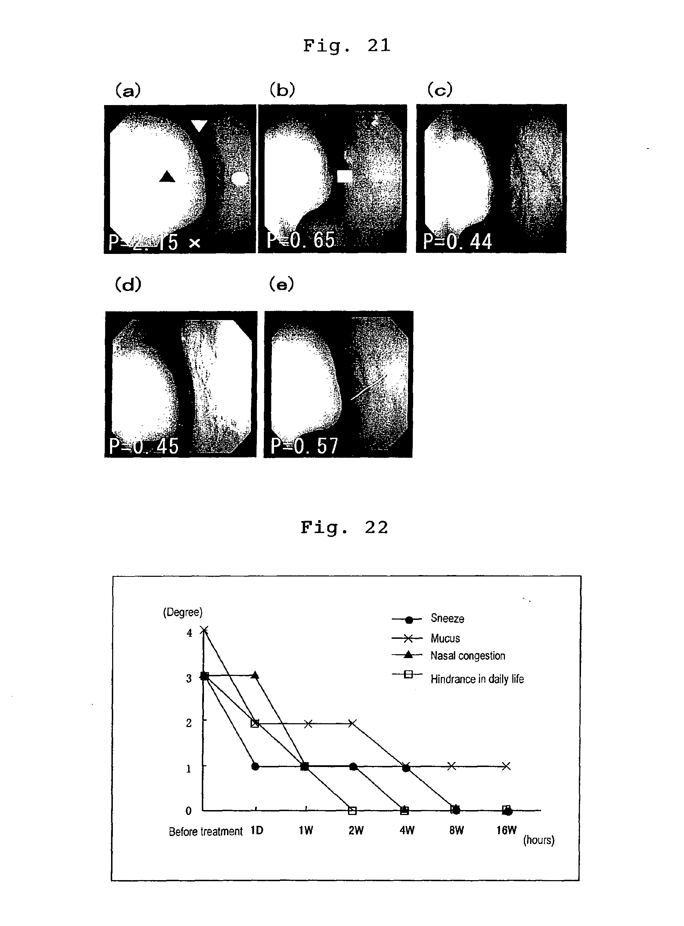

Fig. 20 is a set of photographs showing the observation result of the left inferior turbinate mucosal tissues by a fiber scope before and after the treatment in Example 1-2, wherein (a) is before the treatment, (b) is after 2 weeks, (c) is after 4 weeks, (d) is after 8 weeks, and (e) is after 16 weeks. In Fig. 20, ● shows the nasal septum, ▲ shows the inferior concha, ▼ shows the middle nasal meatus, ■ shows the common nasal meatus, and x shows the inferior nasal meatus, and the part shown by the arrow indicates the mucus. Fig. 21 is a set of photographs showing the observation result of the right inferior turbinate mucosal tissues before and after the treatment in Example 1-2, wherein (a) is before the treatment, (b) is after 2 weeks, (c) is after 4 weeks, (d) is after 8 weeks, and (e) is after 16 weeks. In Fig. 21, ● shows the nasal septum, ▲ shows the inferior concha, ▼ shows the middle nasal meatus, ■ shows the common nasal meatus, and × shows the inferior nasal meatus. P in Figs. 20 and 21 has the same meaning as that of P in Figs. 17 and 18. Fig. 22 is a graph showing the evaluation result based on the evaluation criteria in Table 4 with time after the treatment of the patient in Example 1-2.

### [Results]

As is evident from Fig. 20, the thickening of the left inferior turbinate mucosa and storage of watery rhinorrhea in the common nasal meatus were recognized before the treatment, but after the treatment of the left nasal cavity with the ethanol-containing therapeutic causing contraction of nasal mucosa according to the present invention, the surface of the mucosa was hardly damaged, the mucosa was reliably contracted, and secretion of watery rhinorrhea was hardly recognized, and the effect still lasted even after 16 weeks after the treatment. The left cavity airway resistance was always kept at 1 or less 2 weeks after the treatment and thereafter, thus revealing a constant effect on opening of the nasal cavity. As is evident from Fig. 21, the right nasal cavity subjected to treatment with the ethanol-containing therapeutic causing contraction of nasal mucosa according to the present invention also had the same effect as in the left nasal cavity. The left and right nasal cavity resistance was 0.83 before the treatment, but was reduced to 0.27 in 16 weeks after the treatment.

As is also evident from Fig. 22, sneezes, rhinorrhea and nasal congestion, that is, 3 major allergic symptoms in the patient subjected to the treatment in Example 1-2, were reduced to half in 1 week. Particularly, after 2 weeks, the hindrance in daily life having shown score 3 became 0, and after 4 weeks, any nasal symptoms were reduced to 1 or less. After the treatment in Example 1-2, administration of medicines such as anti-allergic agent and antihistaminic agent including a sedative was not conducted.

Accordingly, it can be said that the effect of the ethanol treatment according to the present invention is equal to or higher than that of the high-frequency tissue reducing method. Further, occurrence of unexpected nasal complications such as infections in the nasal cavity, nosebleed and olfactory disturbance accompanying the treatment using the method of the present invention was not recognized.

### [Example 1-3]

The same treatment as in Example 1-2 was also conducted for other patients (20 persons), whereby very good results similar to those described above were given with very high probability. The results as compared with those of conventional surgical treatment (nasal mucosal resection, laser ablation, cryosurgery) are shown in Table 5.

**(Table 5)**

| Therapeutic method | Reporter | Sneeze (%) | Rhinorrhea (%) | Nasal congestion (%) |
|---|---|---|---|---|
| Therapeutic method of the present invention | Clinical experiment (n=20) | 94 | 87 | 90 |
| Laser operation | average in the past | 64 | 70 | 90 |
| Inferior turbinate mucosa broad resection | average in the past | 56 | 46 | 89 |
| Cryosurgery | average in the past | 85 | 89 | 85 |

Specifically, Table 5 shows the degrees of amelioration of sneeze, rhinorrhea and nasal congestion respectively as the therapeutic results of 20 cases (15- to 45-year-old) with perennial/mixed nasal allergy subjected to the treatment of the present invention, conducted once at 2- to 3-week intervals once to thrice in total. The degree of amelioration of sneeze, rhinorrhea or nasal congestion refers to the probability (%) of cases where the amelioration effect occurred (excluding unchanged and deteriorated cases). During an observation period of 3 to 6 months, oral administration of a general anti-allergic agent including antihistamine and a steroid preparation was not conducted in every case.

From Table 5, the degree of amelioration was 94% for sneeze, 87% for rhinorrhea and 90% for nasal congestion in the therapeutic method using the ethanol-containing therapeutic causing contraction of nasal mucosa according to the present invention. These results are extremely superior to the therapeutic results of the conventional laser operation and inferior turbinatemucosa broad resection. Particularly, an outstanding effect was shown on amelioration of sneeze.

### [Example 1-4]

As is evident from the Examples above, ethanol treatment gives a certain amelioration effect on nasal conditions with low invasion. Now, the clinical progress in the therapeutic method using the therapeutic containing ethanol only as the active ingredient (hereinafter referred to as ethanol treatment) and in the therapeutic method (using the therapeutic containing ethanol and a steroid agent as the active ingredients hereinafter referred to as steroid-containing treatment) was monitored in detail for comparison.

The ethanol treatment was conducted on a 21-year-old woman diagnosed to have perennial allergic rhinitis, and 0.4 ml therapeutic consisting of 70% ethanol was injected into the left nasal cavity in the same manner as in Example 1-1. On the other hand, the steroid-containing treatment was conducted on a 40-year-old man diagnosed to have vasomotor rhinitis, and a therapeutic prepared by adding 0.08 ml of 0.4% Decadoron to 0.4 ml of 70% ethanol was injected into the left nasal cavity in the same manner as in Example 1-1.

### [Evaluation]

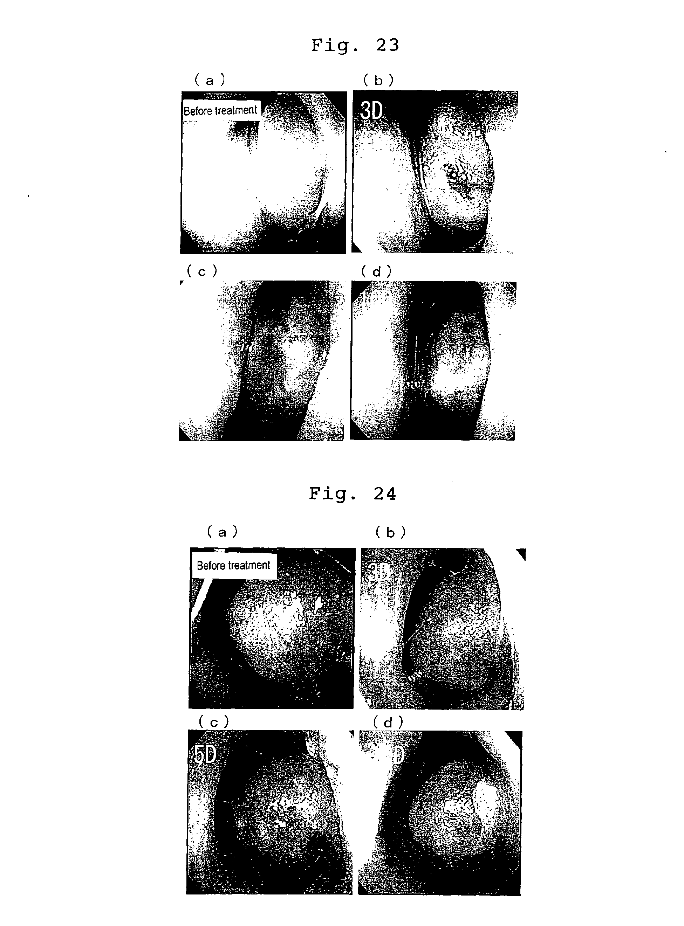

Before and after the ethanol treatment and steroid-containing treatment (after 3 days, after 5 days, after 10 days), the inferior turbinate mucosal tissues were observed with a fiber scope. Fig. 23 is a set of photographs showing the observation result of the left inferior turbinate mucosal tissues by a fiber scope before and after the ethanol treatment in Example 1-4, wherein (a) is before the treatment, (b) is after 3 days, (c) is after 5 days, and (d) is after 10 days. Fig. 24 is a set of photographs showing the observation result of the left inferior turbinate mucosal tissues by a fiber scope before and after the steroid-containing treatment in Example 1-4, wherein (a) is before the treatment, (b) is after 3 days, (c) is after 5 days, and (d) is after 10 days.

### [Results]

As shown in Fig. 23, certain tissue injury occurred for the first 1 to 2 weeks after the ethanol treatment in the epithelial layer of the nasal mucosa due to the permeation of ethanol injected to the submucosa or due to the influence of inflammatory stimulation with ethanol in the ethanol treatment. As shown in Fig. 23(c) and (d), symptoms such as topical erosion of mucosal epithelial layer, ulcer and easy bleeding, which were significantly lower than those by the conventional surgical methods, occurred about 1 week after the treatment.

As shown in Fig. 24, the inflammatory stimulation in the topical tissues, brought about after the treatment, could be minimized in the steroid-containing treatment. As specifically shown in Fig. 23(c), (d) and Fig. 24(c), (d), when the results about 1 week after the ethanol treatment are compared with the results about 1 week after the steroid-containing treatment with a predetermined concentration of steroid added to the same amount and concentration of ethanol, the visual finding of the topical mucosa after the steroid-containing treatment was extremely excellent as compared with that of the ethanol treatment, and damage to the mucosal epithelial layer was hardly observed. That is, it was revealed that the anti-inflammatory action and anti-edema action of the steroid agent were exhibited in the epithelial layer at higher degree than expected, and this method was found to provide a means of easily and reliably inhibiting damage to the epithelial layer to maintain its physiological function at the maximum degree as one important object in the therapeutic method of the present invention, and to be a very effective therapeutic method.

As described above, when the mere effect on contraction of nasal mucosa is to be finally desired, both the ethanol treatment and steroid-containing treatment have a similar equivalent action, but the steroid-containing treatment is evidently superior to the ethanol treatment in that while the invasion into the mucous epithelial layer is further reduced to achieve the very important object to maintain the function of the mucosal epithelial layer, and the clinical therapeutic process is reduced to further improve the therapeutic effect, thus achieving an ameliorating effect on nasal conditions at an early stage in the patient.

### [Example 1-5]

The ethanol treatment and steroid-containing treatment were conducted in 3 cases respectively in the same manner as in Example 1-4, and the change with time in the inferior turbinate mucosa for 2 weeks after the treatment was evaluated on the basis of the following criteria. When reactive swelling or easy bleeding occurred as the inferior turbinate mucosa occupied a larger area of the nasal cavity, and furthermore, evident erosion/ulcerous lesion and scab formation were observed in 50% or more area of the epithelial layer of the inferior turbinate mucosa, +++ (score 3) was given; when some erosion (area less than 10%) remained in the mucosal epithelial layer with contraction of the inferior turbinate mucosa and opening of the nasal meatus, + (score 1) was given; and ++ (score 2) was given between +++ and +. When the damage to the mucosal epithelial layer was very low with slight swelling, ± (score 0.5) was given, and when the mucosal epithelial layer was almost intact and reliable contraction of the mucosal tissues was recognized, - (score 0) was given.

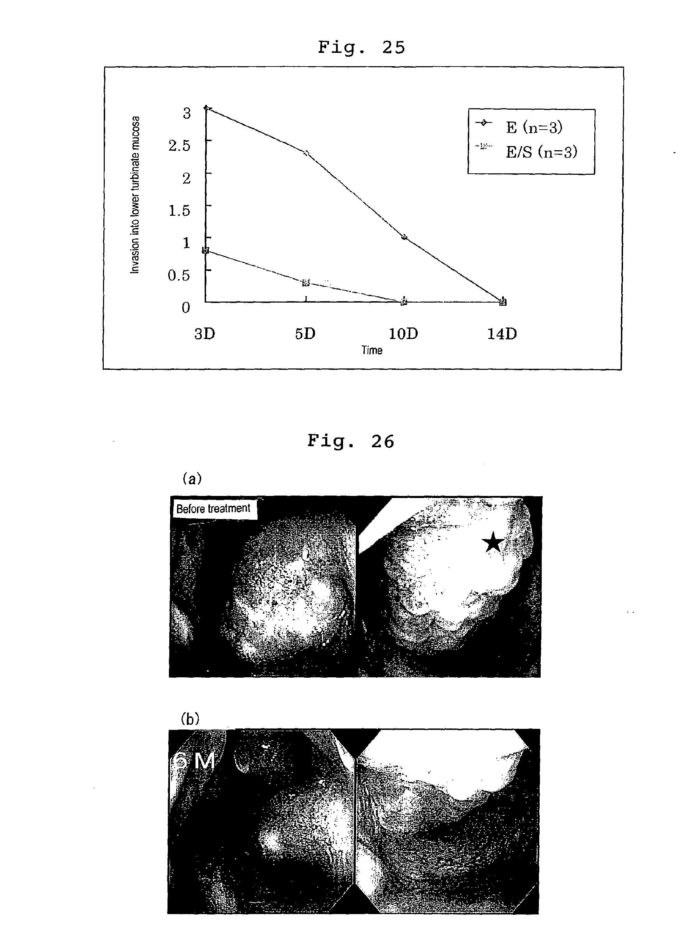

Fig. 25 is a graph showing the degree of invasion (mean) into the inferior turbinate mucosa with time, based on the above evaluation criteria, in 3 patients subjected to the ethanol treatment and steroid-containing treatment in Example 1-5. In Fig. 25, E refers to the ethanol treatment, and E/S refers to the steroid-containing treatment.

As shown in Fig. 25, the steroid-containing treatment is evidently superior to the ethanol treatment in respect of the reduction of the degree of invasion into the nasal mucosa to give an early therapeutic effect.

### [Example 1-6]

### (Therapeutic method)

Under the consent of the patient, the following treatment was carried out. The patient was a 61-year-old man mainly having nosebleed and diagnosed to have chronic rhinitis, deviation of the nasal septum and left nasal polyp (inflammatory polyp). Specifically, deviation of the nasal septum, erythema of the nasal mucosa as a whole, high swelling of the left inferior turbinate mucosa, and papillary growth were recognized by an anterior rhinoscopy in the first diagnosis. In histopathological analysis, biopsy of a grown papillary section revealed nasal polyp. Nasal allergy was negative by examination in serum unspecific/specific antibody assays. In a sinus radiographic examination, a shadow of soft tissues was recognized in the left nasal cavity.

In the treatment, 0.4 to 0.6 ml therapeutic of the present invention consisting of 70% ethanol/0.1% Decadoron was injected into the inferior turbinate submucosa with nasal polyp in left nasal cavity at 3-week intervals thrice in total.

### [Evaluation]

Before the treatment and 6 months after the treatment, the inferior turbinate mucosal tissues were observed with a fiber scope. The inferior turbinate mucosal tissues before the treatment were examined in a histopathological evaluation.

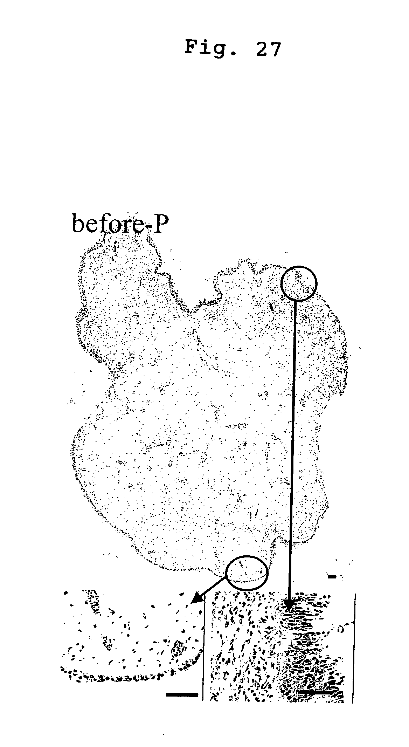

Fig. 26 is a set of photographs showing the observation result of the inferior turbinate mucosal tissues by a fiber scope before and after the treatment in Example 1-6, wherein (a) is before the treatment, and (b) is 6 months after the beginning of the treatment. The left part is weakly magnified, and the right is strongly magnified in the each figure. Fig. 27 is a photograph showing the result of the histopathological examination of the inferior turbinate mucosal tissues before the treatment in Example 1-6.

As shown in Fig. 26(a), examination of the nasal cavity before the treatment indicated non-papillary proliferative mass having small lobulated projections around the left inferior turbinate mucosa. The surface was reddish, easily bled, and accompanied partially by necrosis covered with pale yellow to yellowish brown mossy substance (see 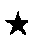 symbol in the figure). As shown in Fig. 27, the histopathological examination before the treatment indicated that the mass was composed mainly of rough connective tissues, and showed intestinal edema, cell infiltration, hyaline thickening of the basement membrane, removal of ciliated columnar epithelium, erosion or degenerative growth, and growth and dilatation of blood vessels, which were in consistent with a pathological image of edema- type nasal polyp. After the treatment, a significant effect on shrinkage of nasal polyp was obtained, and as shown in Fig. 26 (b), the nasal polyp almost disappeared in the nasal cavity 6 months after the beginning of the treatment, and accordingly each nasal meatus was opened, and the whole of the inferior turbinate mucosa resembled that of normal person.

### [Example 1-7]

### (Therapeutic method)

Under the consent of the patient, the following treatment was carried out. The patient was a 52-year-old woman mainly having nasal congestion and rhinorrhea and diagnosed to have allergic rhinitis, deviation of the nasal septum, and nasal polyp (allergic type) in both nasal cavities. Specifically, deviation of the nasal septum, pale swelling in the mucosa in both nasal cavities and secretion of a large mount of watery rhinorrhea were recognized by an anterior rhinoscopy in the first diagnosis. The inferior turbinate mucosa at both sides had edema-like or lobulated polyps, a part of which was partially reddish in outer appearance and rich in blood vessels to cause bleeding easily. Histopathological tissue diagnosis indicated bleeding nasal polyp. Eosinophil examination in the nasal secretion was +++, and in serum-specific antibody assay, the results were cedar (3+), ragweed (3+), and dust (3+), and in the paranasal image analysis and general blood/biochemical examination, there was no abnormality.

In the treatment, 0.4 to 0.6 ml therapeutic of the present invention consisting of 70% ethanol was injected at 3-week intervals thrice in total into the inferior turbinate submucosa with polyp at both sides.

### [Evaluation]

Before the treatment and 8 months after the treatment, the inferior turbinate mucosal tissues were observed with a fiber scope. The inferior turbinate mucosal tissues before the treatment and 6 months after the treatment were subjected to histopathological examination.

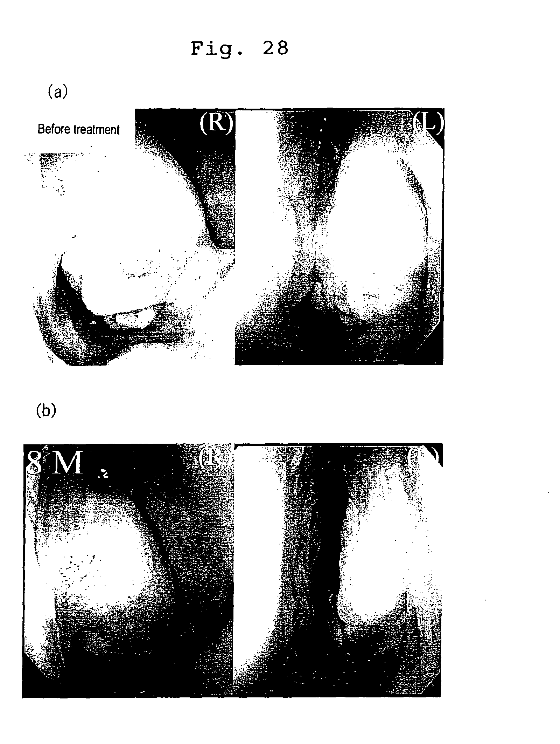

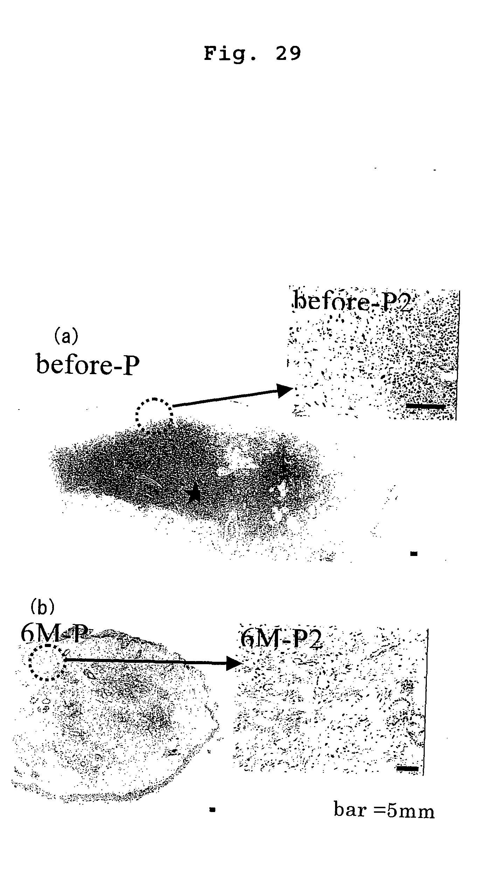

Fig. 28 is a set of photographs showing the observation result of the inferior turbinate mucosal tissues by a fiber scope before and after the treatment in Example 1-7, wherein (a) is before the treatment, and (b) is 8 months after the beginning of the treatment. Fig. 29 is a set of photographs showing the result of the histopathological examination of the inferior turbinate mucosal tissues before and after the treatment in Example 1-7, wherein (a) is before the treatment, and (b) is 6 months after the beginning of the treatment.

### [Results]

As shown in Fig. 28(a), an edematous thickening and lobulated protrusions were recognized in the inferior turbinate mucosa at both sides in diagnosis of the nasal cavity before the treatment. The reddish congestive part of the surface of the mucosa easily bled, and the mucosa had a color of pale red to white as a whole, and a large amount of watery rhinorrhea was observed on the surface. As shown in Fig. 29(a), evident expansion and growth of blood vessels and a focal bleeding plexus (see symbol 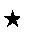 in the photograph) were observed in the edematous interstitium in histopathological examination, and a high degree of infiltration of eosinophils into the interstitium and below the epithelial layer, and exfoliating loss of epithelial cells were recognized, and together with results in other clinical examination, the disease was revealed to be allergic rhinitis with bleeding nasal polyp accompanied by eosinophil infiltration.

After the treatment, the conditions were evidently ameliorated, and as shown in Fig. 29(b), squamous metaplasia of mucosal epithelial cells was observed, but no cellular atypia was recognized in a histopathological image in 6 months after the beginning of the treatment. Further, the edematous change by edema and the focal bleeding plexus in the interstitium in the mucosa disappeared, and the submucosa was reconstructed with newly grown fibers. As shown in Fig. 28(b), the inferior turbinate mucosa at both sides was almost in morphologically normal in 8 months after the beginning of the treatment.

### [Example 2-1]

### (Preparation of therapeutic)

A therapeutic (2 mL) containing 67.5% ethanol and 0.1% Decadoron was prepared by mixing Decadron™ in one tube (2 mg/0. 5 ml, manufactured by Banyu Pharmaceutical Co., Ltd.) with 1.5 mL of 90% ethanol.

### (Therapeutic method)

Under the consent of the patient, the following treatment was carried out. The patient was a 64-year-old man mainly having a symptom of snoring and diagnosed to have snoring of soft palate/uvular vibration type and severe obstructive sleep apnea syndrome. Specifically, snoring of softpalate/uvularvibration type mainly due to a lower position of the soft palate and the excessive length of the uvula was recognized in examinations (X-rays, endoscope etc.) in the first diagnosis, and in a polysomnography examination, respiratory disturbance index AHI (apnea-hyponea index) indicated a severeness of 35.2, and awakening and an evident snoring symptom were also recognized. The score in snore 10-rank evaluation VAS (visual analog scale) by a bed partner was 10.

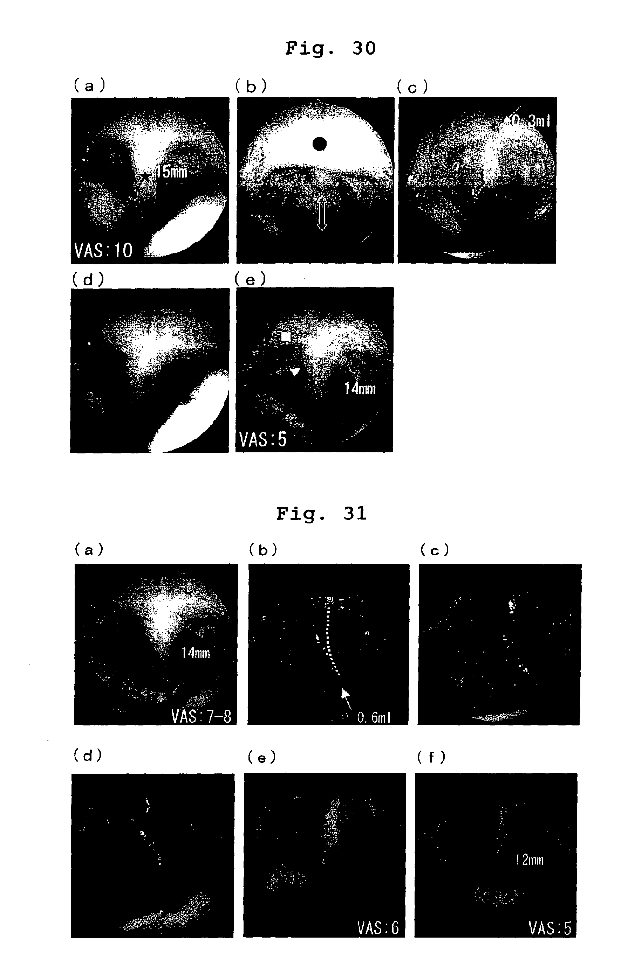

In the pretreatment, the mucosa mainly in the uvular and soft palate was subjected to surface anesthesia with about 5 ml of 4% xylocaine and to infiltration anesthesia with 2 ml of 1% xylocaine E (Astra Zeneca). Then, an injection needle was allowed to pierce into a position by about 0. 7 mm from the center of the radix of the uvula to the side of the soft palate (upward) and into a slightly left position (see arrow in Fig. 30(c)). Then, the injection needle was allowed to go beyond the middle of the axis of the uvula, and 0.3 ml in total of the therapeutic causing contraction of oral pharyngeal mucosal tissues was injected into that position, into the mucosa at the left of uvular radix and in the middle of the uvula respectively while the injection needle was withdrawn, for the purpose to cause contraction of tissues in the uvula and the left palate arch mucosa in the first stage.

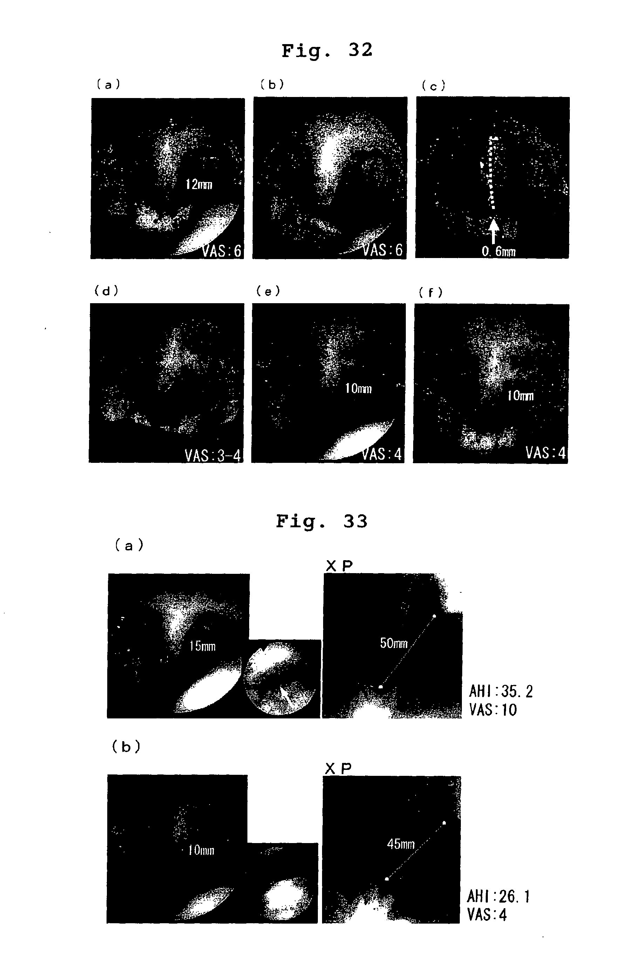

In diagnosis of the oral cavity in 6 weeks after the first treatment, no significant change other than slight shrinkage of the uvula was recognized, but VAS once reduced to 5 after injection of the therapeutic was increased again to 7 to 8, and indicated to increase again, so that in the second treatment, 6 weeks after the first treatment, an injection needle was allowed to pierce into the tip of the uvula (see the arrow in Fig. 31(b)) to inject 0.6 ml of the above therapeutic into the submucosa of the whole of the uvula. In third treatment conducted four months after the first treatment, an injection needle was allowed to pierce into the tip of the uvula (see the arrow in Fig. 32(c)) again, to inject 0.6 ml of the above therapeutic into the submucosa of the whole of the uvula. [Evaluation]

The uvular mucosal tissues before and after the treatment in Example 2-1 were observed by an ocular inspection of the oral cavity and photographed with a fundus camera. Snore 10-rank evaluation (VAS) was also conducted. Fig. 30 is a set of photographs showing the observation result of the uvular mucosal tissues before and after the treatment in Example 2-1, wherein (a) is before the treatment, (b) is at the time of snoring before the treatment, (c) is 1 hour after the first treatment, (d) is 2 weeks after the first treatment, (e) is 4 weeks after the first treatment. Fig. 31 is a set of photographs showing the observation result of the uvular mucosal tissues before and after the treatment in Example 2-1, wherein (a) is 6 weeks after the first treatment, (b) is 30 minutes after the second treatment, (c) is 1 hour after the second treatment, (d) is 3 hours after the second treatment, (e) is 1 week after the second treatment (or 7 weeks after the first treatment), and (f) is 4 weeks after the second treatment (or 10 weeks after the first treatment). Fig. 32 is also a set of photographs showing the observation result of the uvular mucosal tissues in Example 2-1, wherein (a) is 3 months after the first treatment, (b) is 4 months after the first treatment, (c) is 30 minutes after the third treatment, (d) is 1 month after the third treatment (or 5 months after the first treatment), (e) is 6 months after the first treatment, and (f) is 10 months after the first treatment. In Fig. 30, 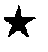 shows the uvula, ● shows the soft palate, ■ shows the anterior palatine arch, and ▼ shows the posterior palatine arch, and the space indicated by the arrow is the pharyngeal cavity, and the point shown by the arrow is a piercing point of the injection needle, and the dotted line is the track of the top of the injection needle. In Figs. 31 and 32, the point indicated by the arrow is a piercing point of the injection needle, and the dotted line is the track of the top of the injection needle.

### [Results]

As is evident from Fig. 30, the length of the uvula was about 15 mm, and the width of the uvula was about 6 mm before the treatment (see (a)), indicating an evident excessive length of the uvula. When the patient was allowed to actually snore in a clinic chair (see (b)), the uvula was completely pulled backwards into the pharyngeal cavity by respiratory pressure, to block the rhinopharyngeal cavity completely, and for not so short while thereafter, the airway in the pharyngeal cavity was completely stopped. One hour after the treatment (see (c)), minor topical swelling and partial whitening of mucosa were observed, but symptoms such as bleeding, a sharp pain and a difficulty in breathing were not observed. Thereafter, the progress was good, and after 2 weeks (see (d), (e)), the whole of the uvula seemed rigid and dense. After 4 weeks, the length of the uvula was rendered shorter by about 1 mm than before the treatment, and the score in snore 10-rank evaluation VAS (visual analog scale) by a bed partner had been reduced to 5.

As shown in Fig. 31, no significant change other than slight shrinkage of the uvula was recognized in examination of the oral cavity in 6 weeks after the first treatment (see (a)), but VAS once reduced to 5 after injection of the therapeuticwas increased again to 7 to 8, indicating that the snore tended to increase again, and thus the second treatment was conducted. As shown in (b) to (d), transient topical swelling of mucosa was observed after the second treatment, but the degree of swelling was gradually decreased with a peak after about 1 hour (see (c)). During this period, symptoms such as a difficulty in breathing were hardly observed. One week after the second treatment, damage to the uvular mucosa was hardly observed with naked eyes (see (e)), and 4 weeks after the second treatment, the length of the uvula was reduced to 12 mm (see (f)). VAS became constantly 6 or less. In the second treatment, the injection volume of the therapeutic was twice as high as that in the first treatment, and the progress after the injection was basically excellent.

As shown in Fig. 32, the uvula was found to be stationary for 3 months and for 4 months after the first treatment (see (a) and (b)). As shown in (c) to (f), the progress was also excellent after the third treatment, and the length of the uvula was reduced finally to 10 mm (width of the uvula, 5 mm) by cicatrical contraction in 10 months after the first treatment. VAS became constantly 4 or less.

Fig. 33 is a set of photographs showing the observation result with a fundus camera upon an ocular inspection of the oral cavity before and after the treatment (6 months after the first treatment), the observation result with a fiber scope partly, the result of cephalometric radiogram (cephalometry), and the results of AHI and VSA. In Fig. 33, (a) is before the treatment, and (b) is 6 months after the first treatment. As shown in Fig. 33, comparison between the results before the treatment and the results in 6 months after the first treatment indicated that after the treatment, the length of uvula was reduced from 15 mm to 10 mm, and it was actually observed under a fiber scope that the protrusion due to the uvula in oropharyngeal cavity (arrow in (a)) was reduced. In a cephalometric radiogram (cephalometry), the length of PNS-P (length of the soft palate: distance from the posterior nasal spine to the tip of the uvula) was also changed from 50 mm to 45 mm. VAS was reduced from 10 to 4, and AHI was also changed from 35.2 before the injection to 26.1.

### [Example 2-2]

### (Preparation of therapeutic)

The therapeutic was prepared in the same manner as in Example 2-1.

### (Therapeutic method)

Under the consent of the patient, the following treatment was carried out. The patient was a 42-year-old man mainly having a symptom of snoring and diagnosed to have a uvula vibration-type simple snore. Specifically, the snore mainly due to excessive length of the uvula and its vibration was recognized in examinations (X-rays, endoscope etc.) in the first diagnosis. In a polysomnography examination, respiratory disturbance index AHI was 1.3 indicating no abnormality, but the score in snore 10-rank evaluation VAS by a bed partner was 7.

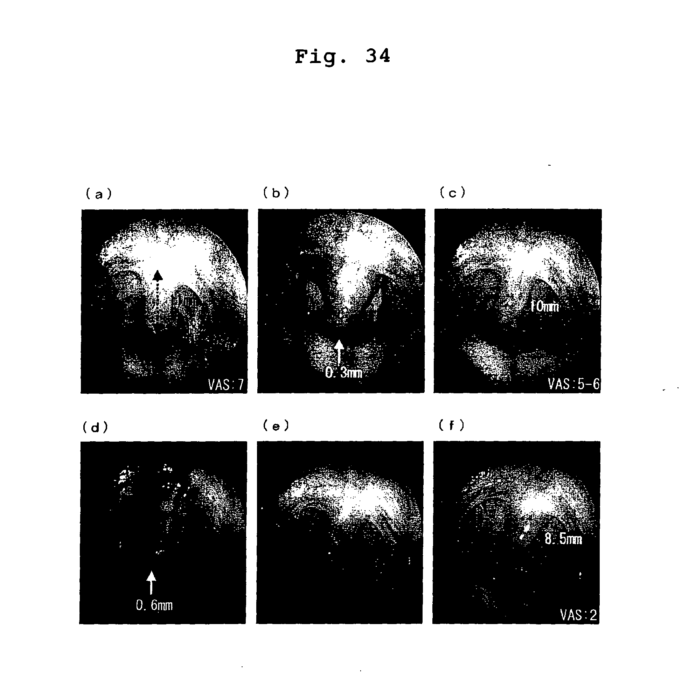

In the pretreatment, the mucosa mainly in the uvula and soft palate was subjected to surface anesthesia with about 5 ml of 4% xylocaine and to infiltration anesthesia with 2 ml of 1% xylocaine E (Astra Zeneca). Then, an injection needle was pierced into the tip of the uvula (see the arrow in Fig. 34(b)), and 0.3 ml of the therapeutic was injected into the submucosa of the whole of the uvula. One month after the first treatment, the uvula was contracted, but because the effect was insufficient, 0.6 ml of the above therapeutic was injected again for the treatment.

### [Evaluation]

The uvular mucosal tissues before and after the treatment in Example 2-2 were observed by an ocular inspection of the oral cavity. Snore 10-rank evaluation VAS was also conducted. Fig. 34 is a photograph with a fundus camera of the uvular mucosal tissues before and after the treatment in Example 2-2, wherein (a) is before the treatment, (b) is 30 minutes after the first treatment, (c) is 1 month after the first treatment, (d) is 30 minutes after the second treatment, (e) is 1 week after the second treatment and (f) is 2 months after the second treatment (or after three months after the first treatment). In Fig. 34, the point indicated by the arrow is a piercing point of the injection needle.

### [Results]

As shown in Fig. 34, the excessive length (11 mm) of the uvula was observed (see (a)) before the treatment, but the uvula was reduced to 10 mm in 1 month after the first treatment (see (c)). After the second treatment, the progress was excellent, and 3 months after the first treatment, the uvula was reduced to 8.5 mm, and VAS was also reduced to 2 (see (e) and (f)). At ten months after the first treatment, the progress was also excellent.

### [Example 2-3]

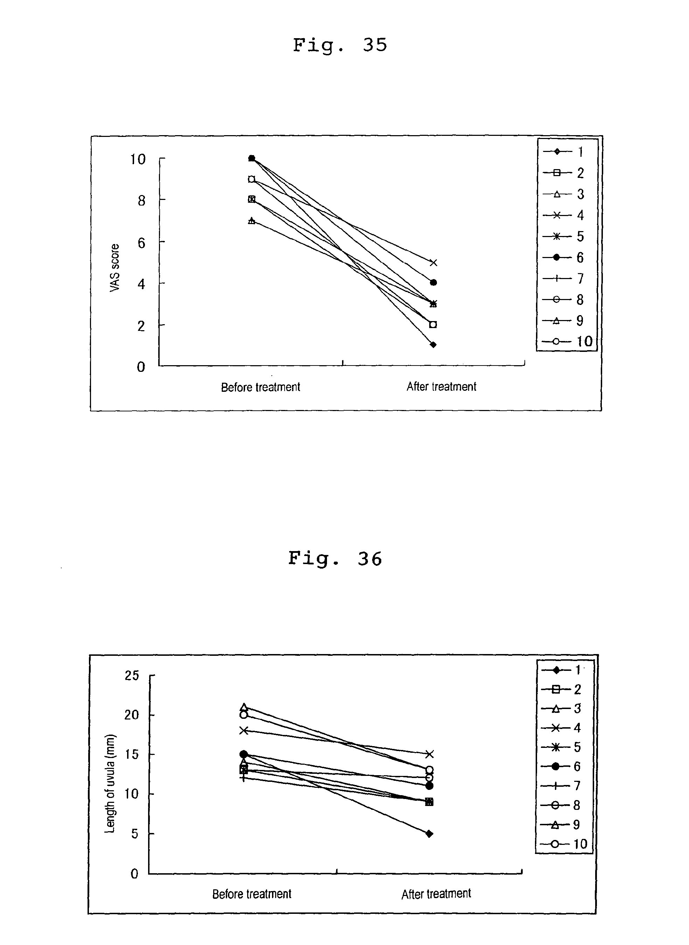

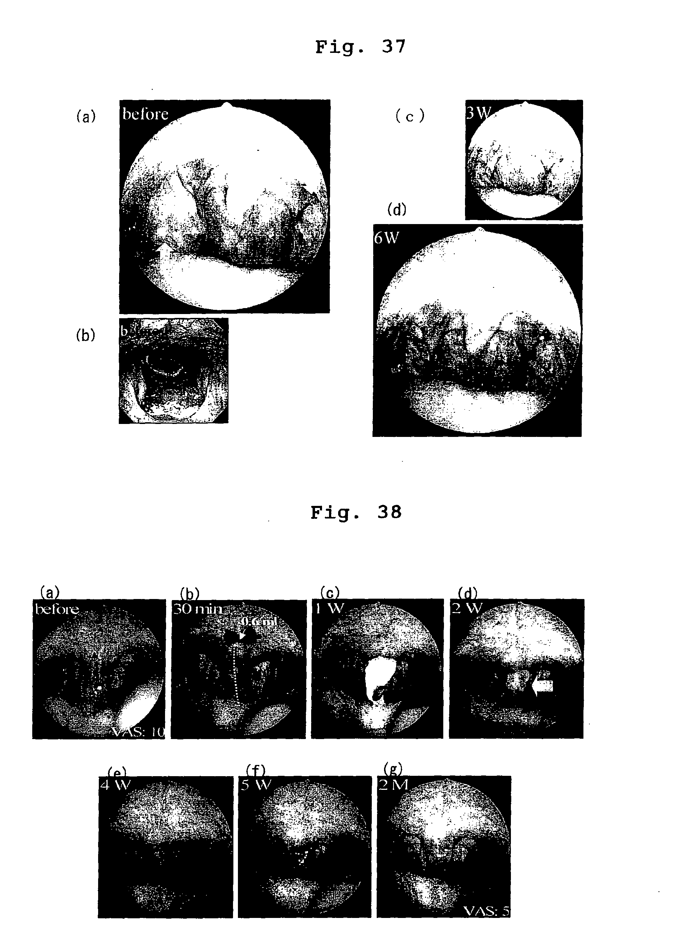

Ten patients with the simple or uvula vibration-type snore (average length of the uvula, 15.9 ± 3.2 mm) having a snore VAS of 7 or more, accompanied by light obstructive sleep apnea syndrome (OSAS) having an AHI of 10 or less, were treated with the therapeutic of the present invention. In all cases, the treatment was conducted once per month or once to thrice (average, 2.4 times) in total, and snore VAS and a change in the length of the uvula were evaluated before the treatment and about 3 to 6 months after the treatment. The results are shown in Figs. 35 and 36.

As is evident from Figs. 35 and 36, the average VAS in all cases was reduced from 8.6 before the treatment to 3.0 after the treatment, and the length of the uvula was also reduced from 15.9 mm before the treatment to 10.3 mm after the treatment, indicating an evident excellent ameliorating effect.

### [Example 2-4]

### (Preparation of therapeutic)

The therapeutic was prepared in the same manner as in Example 2-1.

### (Therapeutic method)

Under the consent of the patient, the following treatment was carried out. The patient was a 2 7-year-old man having repeated tonsillitis and a pharyngeal incongruous sense, and particularly snoring at the acute stage of tonsillitis tended to be worsened, and he was diagnosed to have simple snore of palatine tonsil type and habitual tonsillitis. Specifically, in the observation of the nasal cavity, pharyngeal cavity and oral cavity by an ocular inspection and an endoscope in the first diagnosis, there was no evident abnormality except that the palatine tonsils at both sides showed Mackenzie class II thickening, and lacunar debris partially adhered to the surface. The level in chemical/immune serum assay for ASO (anti-streptlysin O) was as high as 301 (IU/ml). AHI in PSGwas 0.3 indicating no abnormality, but the snore VAS according to a bed partner was 7.

In the pretreatment, the mucosa mainly in the uvula and soft palate was subjected to surface anesthesia with about 5 ml of 4% xylocaine and to infiltration anesthesia with 2 ml of 1% xylocaine E (Astra Zeneca). Then, 0.6 ml of the above therapeutic was injected around the submucosa at the upper extremity of the right tonsil.

### [Evaluation]

The mucosal tissues in the palatine tonsil before and after the treatment in Example 2-4 were examined by an ocular inspection of the oral cavity or by a fiber scope. Fig. 37 is a set of photographs showing the observation result of the mucosal tissues in the palatine tonsil by an ocular inspection of the oral cavity or by a fiber scope before and after the treatment in Example 2-4, wherein (a) is a photograph with a fundus camera upon an ocular inspection of the oral cavity before the treatment, (b) is a photograph by a fiber scope before the treatment, (c) is a photograph with a fundus camera upon an ocular inspection of the oral cavity 3 weeks af ter the treatment , and (d) is a photograph with a fundus camera upon an ocular inspection of the oral cavity 6 weeks after the treatment.

### [Results]

As shown in Fig. 37(a), before the treatment, the palatine tonsils at both sides were exposed from the sinus tonsillaris to the pharyngeal cavity, indicating Mackenzie class II thickening. Storage of yellowish white lacunar debris was recognized in a part of cryptae of the tonsils at both sides (see the arrow in the figure). As shown in Fig. 37(b), the thickening of the tonsils at both sides protruded over the oropharyngeal cavity in the back of the palatopharyngeal arch by diagnosis with a fiber scope. As shown in Fig. 37(c), the volume of the upper extremity of the right tonsil was apparently reduced after 3 weeks, and as shown in Fig. 37(d), the effect was also confirmed after 6 weeks. During the observation of the clinical progress after the treatment, there were none of complications such as pharyngeal infections, acute worsening of tonsillitis, a difficulty in respiration and a difficulty in swallowing.

### [Comparative Example 2-1]

Before the finding of the therapeutic containing the steroid agent in the present invention, the following treatment was conducted under the consent of the patient. The patient was a 53-year-old man having a symptom of snoring, and diagnosed to have the uvula-type snoring accompanied by light sleep respiratory disturbance.

In the pretreatment, the mucosa mainly in the uvula and soft palate was subjected to surface anesthesia with about 5 ml of 4% xylocaine and to infiltration anesthesia with 2 ml of 1% xylocaine E (Astra Zeneca). Then, 0.6 ml of a therapeutic consisting of 70% ethanol in the comparative example was used in treatment by injecting it along the whole length of the uvula.

### [Evaluation]

The uvular mucosal tissues before and after the treatment in Comparative Example 2-1 were observed by an ocular inspection of the oral cavity. Snore 10-rank evaluation VAS was also conducted before the treatment and 2 months after the treatment. Fig. 38 is a set of photographs with a fundus camera of the uvular mucosal tissues upon an ocular inspection of the oral cavity before and after the treatment in Comparative Example 2-1, wherein (a) is before the treatment, (b) is 30 minutes after the treatment, (c) is 1 week after the treatment, (d) is 2 weeks after the treatment, (e) is 4 weeks after the treatment, (f) is 5 weeks after the treatment and (g) is 2 months after the treatment. In Fig. 38(b), the arrow indicates a prickling point of the injection needle, and the dotted line shows a prickling pathway.

### [Results]

As shown in Fig. 38(a), the snore was uvula-type snore accompanied by light sleep respiratory disturbance having a uvula length of about 19 mm (vertical dotted line arrow) and a width of about 8 mm (horizontal dotted line arrow) before the treatment. As shown in Fig. 38(b), there was a possibility that 30 minutes after the treatment, strong damage to the mucosal tissues was caused with a high degree of swelling (the range indicated by the dotted orange line shows the tip of the swollen uvula hidden behind the tong root) of the whole of the uvula, with change of color from grayish violet to dark violet in a part of the mucosa. As shown in Fig. 38(c), diagnosis one week after the treatment indicated that the damage to the mucosa proceeded more than expected, and a majority of the uvular mucosal surface formed white necrotic ulcerous lesions, and particularly the mucosa at its tip was nearly perished and substituted by gray inorganic matter (see the vertical arrow). As shown in Fig. 38(d), the necrotic ulcerous lesions of the uvula tended to be considerably ameliorated (see the horizontal arrow) in 2 weeks, but the perishedmucosa at its tip seemed already removed and disappeared. As shown in Fig. 38(e), after 4 weeks, the ulcerous region of the mucosa was further shrunk, but as shown in Fig. 38(f), the erythema and inflammatory reaction of the topical mucosa did not disappear after 5 weeks. As shown in Fig. 38(g), the mucosal wound of the uvula was almost cured in observation after 2 months, and from the shape, an evident shrinking effect was recognized (length of the uvula, 12 mm), but the deformation of the tip of the uvula still remained.

As described above, the ethanol treatment in the Comparative Example generally showed stronger tissue invasion than in the steroid-containing treatment in the Examples. The time necessary for curing cicatrix in the topical mucosa after the comparative treatment was about 4 to 5 weeks on average (n = 3) including this case, which is at least twice as long as the curing time (about 1 to 2 weeks) after the treatment with the same volume of the steroid-containing ethanol therapeutic.

That is, the actual damaging action on tissues upon using the therapeutic causing contraction of oral pharyngeal mucosal tissues in the Comparative Example was stronger than expected. By the broadening of non-specific inflammatory reaction and invasion to the mucosal surface by ethanol injected into the submucosa, formation of necrotic ulcerous lesions of the mucosa itself tends to be easily caused. Accordingly, it was revealed that after curing of cicatrix, partial defects and deformation of the uvula readily remained. As the curing of tissue damage is prolonged with spread of inflammatory reaction, a longer clinical progress was required until the stable cicatrix shrinking effect was obtained. During the long treatment process, it was revealed that physical and mental pains of the patient, such as a sharp pain and an incongruous sense in the oral cavity and pharynx, a difficulty in swallowing, and poor appetite, are doubled. Further, if the whole of the uvula is not shrunk in a natural form in morphology, to undergo unnatural deformation due to partial structural defects etc., there would be brought about a change in physiological functions in the oral cavity. After surgical treatment such as UPPP (uvulopalatopharyngoplasty) was actually conducted, slight disturbance in voice was actually caused by disturbance of the oral mucosa.

From the foregoing, the therapeutic containing a steroid agent is necessary in treatment of oral pharyngeal mucosal tissues.

### Industrial Applicability

The present invention makes it possible to provide a therapeutic causing contraction of mucosal tissues whereby various diseases relating to mucosal tissues can be easily, safely and little invasively treated, a method of treating various diseases relating to mucosal tissues with the use of the therapeutic causing contraction of mucosal tissues, and an injector and a therapeutic set usable in the treatment method.

### Explanation of Letters or Numerals

10, 50, 70, 90, 104: injector
12, 106: outer cylinder
14, 52, 112: piston
16: side projection member
17: press surface
18, 54: switching mechanism
20, 76, 96: bottom member
22: chemical container
27, 27a: needle member
28, 56, 116: first disk member
30, 58, 118: second disk member
32, 72, 92: cylindrical body
34, 34a: injection needle
35a: inlet
35b: opening
36, 74, 94: stopper member
40: first convex
42: second convex
44, 62: third convex
46, 64: fourth convex
48, 48a: fitting part
60a, 60b, 60c, 60d: tapered member
66: piston pushing member
68: coil spring
86: pin member
78: partition wall
84, 102: break induction part
85, 103: linking part
98: chemical accommodating chamber 99: outer surface
100: supporting member
108, 110: opening window
114: third disk member

## Claims

1. A therapeutic causing contraction of nasal mucosal tissues, which comprises ethanol as an active ingredient.

2. A therapeutic causing contraction of nasal mucosal tissues, which comprises ethanol and a steroid agent and/or an antihistaminic agent as active ingredients.

3. The therapeutic causing contraction of nasal mucosal tissues according to claim 1 or 2, wherein the ethanol is contained in an amount of 30 to 95% by mass.

4. The therapeutic causing contraction of nasal mucosal tissues according to claim 2 or 3, wherein the steroid agent is contained in an amount of 0.01 to 1.0% by mass.

5. The therapeutic causing contraction of nasal mucosal tissues according to any one of claims 2 to 4, wherein the antihistaminic agent is contained in an amount of 0.01 to 1.0% by mass.

6. The therapeutic causing contraction of nasal mucosal tissues according to any one of claims 2 to 5, which is a mixed solution of the active ingredients.

7. A method of treating diseases relating to mucosal tissues, which comprises administering the therapeutic causing contraction of nasal mucosal tissues according to any one of claims 1 to 6 into nasal submucosa.

8. The method of treating diseases relating to mucosal tissues according to claim 7, which comprises direct administration by injection into the nasal submucosa.

9. The method of treating diseases relating to mucosal tissues according to claim 8, wherein the nasal submucosa are inferior turbinate submucosa.

10. The method of treating diseases relating to mucosal tissues according to claim 8, wherein the nasal submucosa is nasal polyp submucosa.

11. The method of treating diseases relating to mucosal tissues according to any one of claims 8 to 10, wherein a predetermined amount of a therapeutic causing contraction of nasal mucosal tissues is injected into a plurality of sites in the nasal submucosa.

12. The method of treating diseases relating to mucosal tissues according to claim 11, which comprises injection into a plurality of sites in a prickling pathway while withdrawing a needle pricked.

13. The method of treating diseases relating to mucosal tissues according to claim 11 or 12, wherein the predetermined amount is 0.05 to 5.0 mL.

14. A therapeutic causing contraction of oral pharyngeal mucosal tissues, which comprises ethanol and a steroid agent and/or an antihistaminic agent as active ingredients.

15. The therapeutic causing contraction of oral pharyngeal mucosal tissues according to claim 14, wherein the ethanol is contained in an amount of 30 to 95% by mass.

16. The therapeutic causing contraction of oral pharyngeal mucosal tissues according to claim 14 or 15, wherein the steroid agent is contained in an amount of 0.01 to 1.0% by mass.

17. The therapeutic causing contraction of oral pharyngeal mucosal tissues according to any one of claims 14 to 16, wherein the antihistaminic agent is contained in an amount of 0.01 to 1.0% by mass.

18. The therapeutic causing contraction of oral pharyngeal mucosal tissues according to any one of claims 14 to 17, which is a mixed solution of the active ingredients.

19. A method of treating diseases relating to mucosal tissues, which comprises administering the therapeutic causing contraction of oral pharyngeal mucosal tissues according to claims 14 to 18 into oral pharyngeal submucosa.

20. The method of treating diseases relating to mucosal tissues according to claim 19, which comprises direct administration by injection into the oral pharyngeal submucosa.

21. The method of treating diseases relating to mucosal tissues according to claim 20, wherein the oral pharyngeal submucosa is uvular submucosa, soft palate submucosa, palatopharyngeal arch submucosa, pharyngeal tonsil submucosa, palatine tonsil submucosa, lingual tonsil submucosa or lateral pharyngeal lymphatic band submucosa.

22. The method of treating diseases relating to mucosal tissues according to claim 20 or 21, wherein a predetermined amount of a therapeutic causing contraction of oral pharyngeal mucosal tissues is injected into a plurality of sites of the oral pharyngeal submucosa.

23. The method of treating diseases relating to mucosal tissues according to claim 22, which comprises injection into a plurality of sites in a prickling pathway while withdrawing a needle pricked.

24. The method of treating diseases relating to mucosal tissues according to claim 22 or 23, wherein the predetermined amount is 0.05 to 5.0 mL.

25. A side successive pushing injector comprising an outer cylinder, a piston capable of moving in the outer cylinder, a side projection member arranged to vigorously project from the side of the outer cylinder to the outside, a switching mechanism engaging with the piston and the side projection member, to advance the piston by pressing the side projection member toward the axis of the outer cylinder, a chemical container accommodating part arranged in the outer cylinder at the advancing side of the piston, to accommodate and retain a chemical container equipped with a bottom member capable of being pushed in a water tight state by advance of the piston, a stopper mechanism preventing return of the advanced piston, and a needle member arranged in the tip of the outer cylinder and equipped with an injection needle.

26. The side successive pushing injector according to claim 25, which is used in treatment of diseases relating to mucosal tissues.

27. The side successive pushing injector according to claim 25 or 26, which further comprises a temperature controlling means.

28. The side successive pushing injector according to any one of claims 25 to 27, wherein a part or the whole of the injection needle is curved.

29. The side successive pushing injector according to claim 28, wherein the direction of the tip of the injection needle, relative to the axial direction of the injection needle, is beyond 0° at 130°.

30. The side successive pushing injector according to any one of claims 25 to 29, wherein the rear of the piston protrudes from the rear of the outer cylinder.

31. The side successive pushing injector according to any one of claims 25 to 30, wherein the outer cylinder is provided with an opening window capable of introducing and removing the chemical container.

32. A therapeutic set comprising the side successive pushing injector according to any one of claims 25 to 31 and a therapeutic-containing chemical container capable of being accommodated in the injector.

33. The therapeutic set according to claim 32, which is constituted such that an amount of 0.01 to 0.2 mL can be jetted out by pushing a side projection member once.

34. The therapeutic set according to claim 32 or 33, wherein the chemical container has a body part, a stopper member and a bottom member, and the body part is provided with a partition wall for separating two chemicals.

35. The therapeutic set according to claim 34, wherein a peripheral region of the partition wall is fixed to the inside of the body part, and a break induction part is formed on the surface of the partition wall.

36. The therapeutic set according to claim 35, wherein the break induction part is a C-shaped linear break induction part.

37. The therapeutic set according to any one of claims 34 to 36, wherein the chemical container has a pin member to break the partition wall with pushing.

38. The therapeutic set according to any one of claims 34 to 37, wherein the partition wall is arranged in the vicinity of the stopper member.

39. The therapeutic set according to claim 32 or 33, wherein the chemical container has a body part, a stopper member and a bottom member, and a chemical accommodating chamber is arranged in the stopper member.

40. The therapeutic set according to claim 39, wherein a supporting member is arranged in the bottom of the chemical accommodating chamber, and a break induction part surrounding the supporting member is formed in the bottom of the chemical accommodating chamber.

41. The therapeutic set according to claim 40, wherein the break induction part is a C-shaped linear break induction part.

42. The therapeutic set according to any one of claims 39 to 41, wherein an injection needle in an injection needle member is arranged with eccentricity, and upon fitting of the needle member to an outer cylinder, the bottom of the chemical accommodating chamber is broken by pressing with the rear end of the injection needle against it.

43. The therapeutic set according to claim 32 or 33, wherein the therapeutic in the chemical container is the therapeutic causing contraction of nasal mucosal tissues according to any one of claims 1 to 6.

44. The therapeutic set according to claim 32 or 33, wherein the therapeutic in the chemical container is the therapeutic causing contraction of oral pharyngeal tissues according to any one of claims 13 to 17.

45. The therapeutic set according to claims 34 to 42, wherein the active ingredients in the therapeutic causing contraction of nasal mucosal tissues according to any one of claims 2 to 5 are accommodated separately.

46. The therapeutic set according to claims 34 to 42, wherein the active ingredients in the therapeutic causing contraction of oral pharyngeal mucosal tissues according to any one of claims 13 to 16 are accommodated separately.
